(19) Europäisches Patentamt
European Patent Office
Office européen des brevets

(11) EP 0 262 343 B1

(12) EUROPÄISCHE PATENTSCHRIFT

(45) Veröffentlichungstag und Bekanntmachung des Hinweises auf die Patenterteilung: **03.01.1996 Patentblatt 1996/01**

(21) Anmeldenummer: **87111381.7**

(22) Anmeldetag: **06.08.1987**

(51) Int. Cl.[6]: **A61K 47/00**, A61K 9/10, C07D 213/00, C07D 239/00, C07D 241/00, C07D 473/00, C07D 209/00, C07D 231/00, C07D 233/00, C07D 236/00, C07D 277/00

(54) **N-alkylierte quartäre stickstoffhaltige Heterozyklen, Verfahren zu deren Herstellung und deren Verwendung in Arzneimitteln**

N-alkylated quaternary nitrogen-containing heterocycles, process for their preparation and their use in pharmaceutical compositions

Hétérocycles azotés N-alkylés quaternaires, procédé pour leur préparation et utilisation en compositions pharmaceutiques

(84) Benannte Vertragsstaaten:
**BE CH DE FR GB IT LI LU NL SE**

(30) Priorität: **07.08.1986 DE 3626700**

(43) Veröffentlichungstag der Anmeldung: **06.04.1988 Patentblatt 1988/14**

(73) Patentinhaber: **MEDICE Chem.-Pharm. Fabrik Pütter GmbH & Co. KG D-58638 Iserlohn (DE)**

(72) Erfinder: **Hasko, Henrich Prof.Dr.med.Dr.rer.nat. D-5860 Iserlohn (DE)**

(74) Vertreter: **Reinhard - Skuhra - Weise & Partner D-80750 München (DE)**

(56) Entgegenhaltungen:

| | |
|---|---|
| **EP-A- 0 004 368** | **EP-A- 0 161 987** |
| **CH-A- 177 815** | **DE-B- 1 300 948** |
| **DE-C- 820 949** | **FR-A- 1 435 785** |
| **FR-A- 1 548 252** | **US-A- 2 056 449** |
| **US-A- 2 643 967** | **US-A- 2 759 821** |
| **US-A- 2 782 204** | **US-A- 3 911 133** |

- **JOURNAL OF PHARMACEUTICAL SCIENCES, Band 71, Nr. 12, Dezember 1982, Seiten 1313-1318, Am. Pharm. Ass., Washington, US; A. TSUJI et al.: "Effects of surfactants on the aqueous stability and solubility of beta-lactam antibiotics"**
- **ACCOUNTS OF CHEMICAL RESEARCH, Band 12, Nr. 4, April 1979, Seiten 111-118, Am. Chem. Soc., Washington, US; F.M. MENGER: "On the structure of micelles"**
- **JOURNAL OF THE AMERICAN CHEMICAL SOCIETY, Band 108, Nr. 11, Mai 1986, Seiten 2980-2984, Am.Chem. Soc., Washington, US; F.M. MENGER et al.: "A microscopichydrophobicity parameter"**
- **CHEMICAL ABSTRACTS, Band 45, Nr. 12, 25. Juni 1951, Spalten 5099i-5101d, Columbus, Ohio, US; F. TANAKA: "Invert soaps as disinfactants. VIII. Banzimidazolium salts"**
- **AUSTRALIAN JOURNAL OF CHEMISTRY, Band 35, 1982, Seiten 525-534, CSIRO, East-Melbourne, AU; M. RASMUSSEN et al.: "Heterocyclic ambident nucleophiles. III. The alkylation of sodium adenide"**
- **JOURNAL OF THE AMERICAN CHEMICAL SOCIETY, Band 81, Nr. 12, 20. Juni 1959,Seiten 3046-4051, Am. Chem. Soc., Washington, US; H.C. KOPPEL et al.:"Potential purine antagonists. XIX. Synthesis of some 9-alkyl(aryl)-2-amino-6-substituted purines and related v-triazolo(d)pyrimidines"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 262 343 B1

Printed by Rank Xerox (UK) Business Services
2.8/3.4

- JOURNAL OF THE AMERICAN CHEMICAL SOCIETY, Band 85, Nr. 22, 20. November 1963, Seite 3719, Am. Chem. Soc., Washington, US; N.J. LEONARD et al.: "The synthesis of 7-substituted adenines through the use of a blocking group at the 3-position. Site of alkylation of 7-substituted adenines"
- CHEMICAL ABSTRACTS, Band 60, Nr. 5, 2. März 1964, Spalten 5668e-5669a,Columbus, Ohio, US; A.I. KIPRIANOV et al.: "Cyanine dyes from 2-methyl-5,6-dihydroxy- benzothiazole"
- RESEARCH DISCLOSURE, Nr. 140, Dezember 1975, Seiten 33-36, Ref. Nr. 14007, Ind. Opportunities Ltd, Havant, GB; J.D. BASS: "Lithographic fixing compositions"
- HELVETICA CHIMICA ACTA, Band 64, Fasc. 6, 23. September 1981, Seiten 1943-1948, ref.nr.186; Schweizerische Chem. Ges., Basel, CH. E.Barni et al.: "Synthesis surface activity and micelle formation of novel cyanine dyes"
- CHEMICAL ABSTRACTS, Band 99, Nr. 25, 19. Dezember 1983, Seite 393, Ref. Nr. 209667m, Columbus, Ohio, US; P. FOLTINOVA et al.: "Antimicrobial effectivenessof bezothiazolium salts"

## Beschreibung

Die vorliegende Erfindung betrifft N-alkylierte quaternäre stickstoffhaltige Heterozyklen sowie Verfahren zu ihrer Herstellung. Die Erfindung betrifft weiterhin pharmazeutische Zubereitungen, die eine oder mehrere dieser N-alkylierten quaternären stickstoffhaltigen Heterozyklen als eigenständige pharmazeutische Wirkstoffe enthalten sowie pharmazeutische Zubereitungen, die eine oder mehrere dieser N-alkylierten quaternären stickstoffhaltigen Heterozyklen als micellare oder vesikuläre Strukturen enthalten, in die ein oder mehrere hydrophobe pharmazeutische Wirkstoffe eingeschlossen sind. Weiterhin betrifft die Erfindung die Verwendung dieser Heterozyklen als spektroskopische Marker sowie zu kolloid-chemischen Meßverfahren zur Bestimmung der KMK.

Stand der Technik:

Bekannt sind die quartären Ammoniumbasen mit tensidartiger Wirkung der allgemeinen Struktur (I)

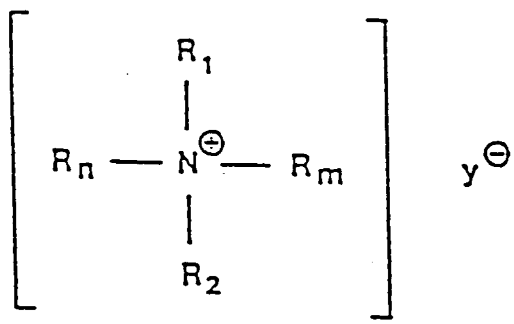

wobei im allgemeinen

| | |
|---|---|
| $R_1$ | = ein Alkylrest mit 1 - 12 C-Atomen |
| $R_2$ | = ein Alkylrest mit 1 - 12 C-Atomen |
| $R_n$ | = ein geradkettiger oder verzweigter Alkylrest mit 10 - 20 C-Atomen oder ein Alkenylrest mit 8 - 10 C-Atomen oder ein 5- oder 6-gliedriger aromatischer Heterozyklus mit einem oder 2 Stickstoffatomen und |
| $R_m$ | = ein geradkettiger oder verzweigter Alkylrest mit 10 - 20 C-Atomen oder ein Alkenylrest mit 8-10 C-Atomen oder ein 5- oder 6-gliedriger aromatischer Heterozyklus mit einem oder 2 Stickstoffatomen |
| $y^-$ | = ein einwertiges Anion |

ist.

Verbindungen dieser allgemeinen Formel sind zum Teil im Tensid-Taschenbuch, herausgegeben von Dr. H. Stache, Carl-Hauser-Verlag, München Wien, 1981, Seite 8/9, beschrieben worden.

Auch sind Teile dieser Verbindung Gegenstand einer europäischen Patentanmeldung, Anmelde-Nr. 83810338.0 vom 24.07.1983, wobei diese Tenside zur Herstellung von unilamellaren Liposomen in wäßriger Phase zur Dispergierung angewendet werden.

Hierbei ist zu berücksichtigen, daß diese bekannten $N^+$-Tenside der allgemeinen Formel I sowohl micellare als auch vesikuläre Strukturen in wäßrigen und unpolaren Lösungsmitteln bilden (siehe z.B. J. Fendler, Acc. Chem. Res. 1976, 9, 153; H.H. Paradies, J. Phys.Chem.1986, 90, 5956; H.H. Paradies, 1982, Angew. Chem 10, 737; Angew. chem. Int. Ed. Engl., 1982, 21, 765, Supplement 1982, 1670-1681) und hier auch definierte, je nach Aufgabenstellung, bestimmte chemische und biophysische Reaktionen, micellar katalysieren.

Dagegen sind kationische Tenside mit einem quartären Stickstoff innerhalb eines $\pi$-Überschuß oder $\pi$ - Mangel-Aromaten, substituiert oder nicht im Kern substituiert, weniger gut bekannt. Es liegen Beschreibungen, z.B. für Hexadecyl-pyridinium-Halogenid (Cetylpyridinium-Halogenid), siehe u.a. Merck-Index 9, Chinolin-, (siehe K. Lindner, in Tenside-Textilhilfsstoffe-Waschrohstoffe (1964, Bd. 1, 987) und für Benzthiazolium-salze (Demande de Brevet Europeen 85400876.0 vom 06.05.1987 sowie BE 660.802 vom 08.03.1965) vor, und zwar mit verschiedenen Alkylkettenlängen und Gegenionen mit Anwendungen in der Photographie und Elektronenübertragung durch geeignete Bildung von Charge-Transfer-Komplexen. Es handelt sich hier allerdings um 2-Methyl bzw. 2-substituierte Benz-thiazolium-Verbindungen mit variabler hydrophober Alkylkettenlänge von 12 - 30 Kohlenstoffatomen am Heterozyklus des ankondensierten Benzolringes.

Weiterhin sind nach dem Stand der Technik die 2-substituierten Imidazoliumsalze und 2-Thiazolium-Verbindungen beschrieben (siehe Tensid-Taschenbuch, H.Stache, 2. Auflage, 1981, Seite 8/9), ohne allerdings KMK und andere micellare Eigenschaften anzugeben. Entsprechendes ist auch für die Imidazoliumverbindungen beschrieben worden, siehe z.B. Tensid-Textilhilfsmittel-Waschrohstoffe- K. Lindner, 1964, 993; wissenschaftlicher Verlagsgesellschaft, Stuttgart.

Für vesikuläre Verbindungen mit einem Pyridinring als Aromaten sind nur 4- bzw. 3,5 Alkyl bzw. Alkoxyl-Verbindungen beschrieben worden, welche am quatären Stickstoff eine Methylgruppe enthalten (siehe z.B. Sudhölter et al. 1982, J. Amer. Chem. Soc. 104, 1069, Sudhölter et al. 1979, J. Amer. Chem. Soc. 102, 2467).

Research Disclosure December 1975, Nr. 14007 offenbart lithographische Fixiermittelzusammensetzungen mit 7-Dodecylbenzthiazoliumiodid und -bromid.

EP 0 161 987 offenbart N-Octadecylbenzthiazoliumiodid. US-2 759 821 offenbart 3-n-Laurylbenzdiazoliumiodid. US-2 056 449 offenbart Cetylbenzimidazoliumchlorid und 7-Dodecylchloridhydroxymethylbenzimidazoliumchlorid. US-3 911 133 offenbart 1-n-Decylbenzimidazoliumbromid und 1-n-Octyl-benzimidazoliumbromid.
CA-1951, Vol. 45, Spalte 5100, Teil VIII offenbart 1-Dodecylbenzimidazoliumchlorid.

Es ist eine Aufgabe der vorliegenden Erfindung, neue N-alkylierte quartäre stickstoffhaltige Heterozyklen bereitzustellen.

Diese Aufgabe wird erfindungsgemäß dadurch gelöst, daß folgende Verbindungen bereitgestellt werden:
N-alkylierte quartäre stickstoffhaltige Heterozyklen der allgemeinen Formel

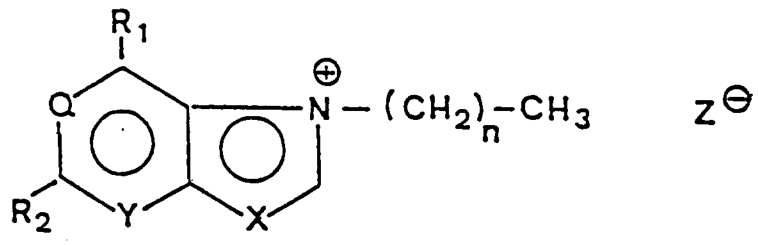

X = NH ; S
Y = N ; CH
Q = N ; C-$CH_3$ ; CH
$R_1$ = H ; OH ; F ; $NH_2$
$R_2$ = H ; OH ; $NH_2$
n = 8 bis 20 insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$, ausgenommen die folgenden Verbindungen: 7-Dodecylbenzthiazoliumiodid und -bromid,
N-Octadecylbenzthiazoliumiodid,
Cetylbenzimidazoliumchlorid,
7-Dodecylchloridhydroxymethylbenzimidazoliumchlorid,
1-n-Decylbenzimidazoliumbromid,
1-n-Octyl-benzimidazoliumbromid,
1-Dodecylbenzimidazoliumchlorid.

Vorzugsweise Ausführungsformen sind folgende Verbindungen, wobei bei den angegebenen Strukturformeln jeweils $Z^-$ die vorstehenden Anionen und n= 8 - 20 bedeuten:

1. 7-n-Alkyl-imidazolium [4,5-d]-pyrimidin der Formel

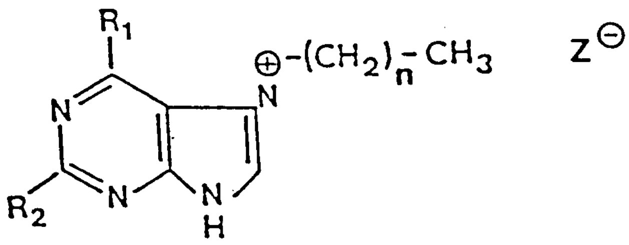

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$
wobei $Z^-$ die vorstehenden 17 Anionen und n 8 - 20 bedeuten.

2. 7-Hexadecylimidazolium [4,5-d]pyrimidin der Formel

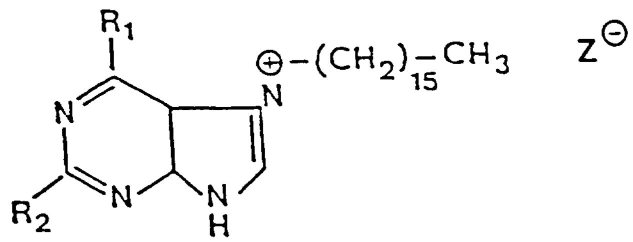

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$
wobei $Z^-$ die vorstehenden 17 Anionen bedeuten.

3. Weiterhin offenbart die Erfindung 3-n-Alkyl-5,6-substituierte Benzimidazolium-Verbindungen der Formel

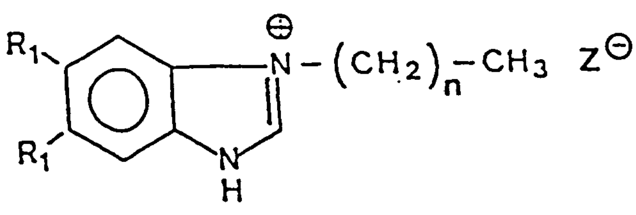

$R_1$ = OH
wobei $Z^-$ die vorstehenden 17 Anionen und n 8 - 20 bedeuten, und

4. 3-n-Alkyl-5,6-substituierte Benzthiazolium-Verbindungen der Formel

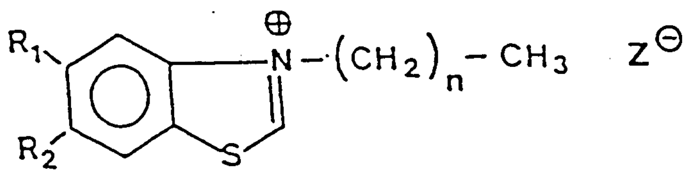

$R_1 = R_2 = OH$
$R_1 = R_2 = CH_3$
wobei $Z^-$ die vorstehenden 17 Anionen und n 8 - 20 bedeuten.

Herstellung der erfindungsgemäßen n-alkylierten quaternären stickstoffhaltigen Heterozyklen:

a.) Allgemein zur Herstellung:

Diese kationischen Tenside sind dadurch charakterisiert, daß sie eine sehr kleine Micellbildungskonstante (KMK) von ungefähr $1{,}0 \cdot 10^{-6}$ - $1{,}5 x 10^{-7}$ Mol/Liter haben, sehr stark antimikrobiell und antifungal wirksam sind, keine Polydispersität in Gegenwart von anorganischen Anionen bzw. Potenzierungsgemischen zeigen, und z.T. selbst mikrobielle Stoffwechselprodukte (Antimetabolite) sind, die nicht toxisch für die Wirtszelle sind.

Die Ausbildung der salzartigen Struktur dieser Klasse von kationischen Tensiden der Form ($HET{\equiv}N^+$-$(CH_2)_x$-$CH_3$) $Y^-$ ist u.a. in der Elektronendichte-Verteilung der heteroaromatischen Kerne bzw. in ihrer Basizität, einschließlich des Einflußes der Substituenten, begründet. Eine notwendige Bedingung, welche zur Ausbildung von quartären Salzen dieser heteroaromatischen Klasse führt, besteht darin, daß die Elektronendichte an Stickstoff, welcher quartärniert wird, nach MO-SCF-Rechnungen einen Betrag von -0,08 bis -0,159 haben muß. Diese Stabilität der einzelnen hier beschriebenen heterozyklischen kationischen Tenside wird außerdem noch durch ihre Symmetrie und Kettenlänge der Alkylkette am quartären Stickstoff bestimmt:
Die Länge der Alkylkette am quartären Stickstoffatom bestimmt nicht nur Schmelzpunkt und Hydrophobizität der später in wäßrigen Lösungen gebildeten kationischen Micellen, sondern auch die Ausbeuten nehmen mit zunehmender Kettenlänge ab, während die Reaktionszeiten z.B. in Nitrobenzol oder 2-Ethoxyethanol zunehmen.

Stabile und leicht kristallisierbare Verbindungen werden für $C_{12}$-$C_{18}$ erhalten, wobei das Gegenion $Y^-$ ausnahmslos Bromid und Chlorid ist. Die anderen Verbindungen können leicht aus Aceton oder Chloroform umkristallisiert werden. Die entsprechenden Jodverbindungen sind temperatur- und lichtempfindlich.

b) Speziell zur Herstellung

Herstellung von 7-n-Alkyl-imidazolium [4,5-d] Pyrimidin-Salzen:

Die Verbindungen gemäß der Überschrift lassen sich auf zwei verschiedenen Synthesewegen herstellen:

1. Durch Herstellung, z.B. ausgehend von Adenin, des Natriumsalzes des Adenins oder Purins mit anschließender Alkylierung durch die entsprechenden n-Alkyl-Halogenide, insbesondere von n-Alkyl-Bromiden.
Die Herstellung des Natriumsalzes der allgemeinen Formel

(I)

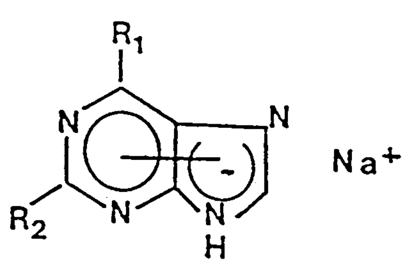

erfolgt durch Umsatz der unsubstituierten bzw. substituierten, d.h. $R_1 = R_2 = H$; oder $R_1 = OH$; $R_2 = OH$; $R_1 = F$, $R_2$

= $NH_2$; $R_1$ = F, $R_2$ = OH; $R_1$ = $NH_2$, $R_2$ = H; $R_1$ = $NH_2$; $R_2$ = $NH_2$ Verbindungen der Formel (I) mit Natriumhydrid in N,N-Dimethylformamid. Dieses Verfahren, insbesondere in Gegenwart der aufgezählten Substituenten, erhöht die Selektivität der Alkylierung nach geeigneten Lösungsmittelbedingungen der 7-Positionen gegenüber den entsprechenden Isomeren der 9-Positionen.

Dieser Syntheseweg gewährleistet außerdem eine gezielte Substitution in der 7-Positionen in Bezug auf 7-(ω-halogen-alkyl)-Purine, substituiert oder nicht substituiert, z.B. auch von 7-(ω-substituierte-alkyl)-Adeninen.

2. Durch gleichzeitigen Umsatz von Triethyloxoniumbor-tetra-fluorid ($Et_3O^+BF_4$) und n-Alkylbromid in Aceton oder Nitrobenzol mit Verbindungen der allgemeinen Formel II

(II) 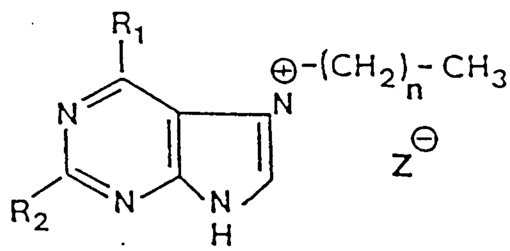

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$

mit $R_1$ = H; OH; F; $NH_2$ und $R_2$ = H; OH; $NH_2$.

Zur Erhöhung der Ausbeuten ist es bei Anwendung dieser Methode nötig, die Hydroxyl-Gruppen durch übliche Schutzgruppen, z.B. durch Benzyl- oder Tritylreste, zu schützen und später zu hydrolysieren, ohne daß die n-Alkylgruppe an Position 7 davon betroffen wird.

a) Herstellung der Natriumsalze von (I) oder z.B. von Adenin:
Eine Suspension von 0,20 Mol (= 25 g Adenin z.B) und 0,22 Mol (= 5,1 g Natriumhydrid im Falle von Adenin) werden in 500 ml von wasserfreiem N,N-Dimethylformamid bei 20°C unter stetem Rühren eingetragen. Das Natriumhydrid ist vorzugsweise in wasserfreiem Mineralöl dispergiert. Nach einer Stunde entwickeln sich die entsprechenden Natriumsalze der Formel (I). Diese so erhaltene Suspension wird für die Alkylierung benutzt.

b) Vorzugsweise wird die Alkylierung in Nitrobenzol vorgenommen. Zu dieser, vorne genannten Suspension (a) gibt man 100 ml wasserfreies Nitrobenzol und tropfenweise unter steter Temperaturkontrolle bei 190°C 0,25 Mol n-Alkylbromid zu. Die Reaktion wird für die Dauer von 24 Stunden unter stetem Umrühren durchgeführt. Die entsprechenden 7-N-Alkylimidazolium-[4,5-d]-Pyrimidin-Salze präzipitieren nach Abkühlung der Reaktion aus. Umkristallisiert wird aus absolutem Ethanol oder wasserfreiem Chloroform. Es werden farblose Nadeln erhalten, die für n= 10 - 20 ($CH_2$) Ausbeuten von nahezu 35 % ergeben, und gemäß nachfolgender Aufstellung folgende Schmelzpunkte haben:

| n | 7-n-Alkyl-adenin-Salze | Fp (°C) |
|---|---|---|
| 8 | 7-Octyladenin-bromid | 204 |
| 9 | 7-Nonyl-adenin-chlorid | 194 |
| 10 | 7-Decyl-adenin-chlorid | 162 |
| 15 | 7-Pentadecyl-adenin-chlorid | 160 |
| 16 | 7-Hexadecyl-adenin-chlorid | 130 |
| 20 | 7-Dodecyl-adenin-chlorid | 125 |

Weitere, neuere n-Alkyl-verbindungen der Purine sind in Tabelle 1, deren physikalischchemische Eigenschaften in Abhängigkeit von $Z^-$, in Tabelle 2 aufgeführt.

$\lambda_{max}$ pH 7,0 dieser Verbindungen liegen bei 272-274 nm; die Verbindungen der Adenin-Klasse bei $\lambda_{max}$, pH 1,0 bei 269 nm, pH 7,0, 272 nm; $R_f$ 0,46 in 1:1 Ethanol-Chloroform auf Silika-Gel.

c. Herstellung der 7-n-Alkyl-imidazolium [4,5-d] pyrimidinderivate (Purin), z.B. 7-Hexadecylimidazolium-2,6-dihydroxy [4,5-d] pyrimidinbromid:
1,5 g 2,6-Dihydroxy-purin (0,01Mol) werden in 100 ml Aceton im Vierhalskolben bei 35°C gelöst. Aus zwei Tropftrichtern werden unter Rühren und Stickstoff einmal Triethyl-oxonium-bortetra-fluorid ($Et_3O^+BF_4$) in dreifachem Überschuß (5,7 g = 0,03 Mol) gegenüber n-Hexadecylbromid (3,3 g, 0,01 Mol), das sich in dem zweiten Tropftrichter befindet, gleichzeitig mit n-Hexa-decyl-Br zugetropft. Die Reaktion wird unter stetem Rühren über 6 Stunden bei 40°C gehalten und anschließend 10 Stunden bei 65°C am Rückfluß gekocht. Nach Abschluß der Reaktion setzt man 100 ml Ethanol zu, filtriert die gebildete quartäre Ammoniumbase über einen Glassintertiegel (1G4) ab und kristallisiert aus einem Gemisch, bestehend aus 2-Ethoxyethanol/Chloroform, 1:1 um . Ausbeute: 0,5 g, Schmelzpunkt 122°C.

Die Verbindung ist hygroskopisch und bildet ein kristallines Addukt mit zwei Teilen Chloroform (Tabelle 1 und 2).
Die UV-Spektren zeigen die typischen Absorptionseigenschaften der Purinderivate. Desgleichen die $^1$H-NMR-Spektren, gemessen in $d_6$-$Me_2SO_4$.

d) Die entsprechenden Benzothiazol- und Benzimidazol-n-Alkyl-Verbindungen, vor allem wenn sie in der 2-Stellung halogeniert sind, bilden sich nach diesem Verfahren mit einer Ausbeute von 50 % und sind sehr leicht aus Chloroform umzukristallisieren (Tabelle 1 und 2).

Charakteristisch für alle erfindungsgemäß hergestellten 7-n-Alkyl-imidazolium [4,5-d] pyrimidin-Salze, insbesondere der Bromide und primären Phosphate, ist der Einfluß der ersten Methylengruppe am N(7) auf das Ringproton im $^1$H-NMR-Spektrum: es wird eine zu kleineren Feldstärken chemische Verschiebung durch die Ausbildung des quaternären Stickstoffes N(7) beobachtet, zugleich mit einer beträchtlichen Entkoppelung des Protons am C (8), mit dem Ergebnis, daß die Versetzung eine Verschiebung von $\delta$ 1,3 ppm im Verhältnis zu $\delta$ 8,3 ppm am C(8) bewirkt. Daher erscheint dieser Peak des Protons am C (8) dieser Salze als ein scharfes Singlet bei $\delta$9,6 ppm im $^1$H-NMR-Spektrum in Lösungen von Methyl-sulfoxid-$d_6$. Darüber hinaus erscheint ein schneller Austausch dieses C (8)-Protons nach Zugabe von $D_2O$, so daß der Peak bei $\delta$ 9,6 ppm verschwindet.
Entsprechendes wird für die erfindungsgemäß hergestellten $N^+$-Tenside der substituierten Benzimidazole und Thiazole beobachtet.
In wäßrigen Lösungen unter pH 7,0 (pH 3,9 - 5,9) zeigen sich bei dieser Micellengröße von unter 600 Å im Durchmesser die typischen scharfen Signale bei $\delta$ ca. 0,85 - 0,89 ppm ($-CH_3$), $\delta$ ca. 1,28 - 1,30 ppm ($-Ch_2-$) und $\delta$ ca. 3,25 ppm ($n-CH_2-$) für diese $N^+$-Tenside zusätzlich zu den Protonen-Kopplungssignalen des substituiertenn bzw. unsubstituierten $\pi$-Überschuß-Aromaten-Systems der erfindungsgemäß hergestellten $N^+$-Tenside.
Tabelle 1 zeigt einige charakteristische Verbindungen aus der Klasse der erfindungsgemäßen Heterozyklen.
Tabelle 2 zeigt die physikalisch-chemischen hydrodynamischen Radien in Abhängigkeit von $Z^-$.

Tabelle 1

Charakteristische Eigenschaften der hergestellten Substanzen

| Nr. | N-Tensid | $Z^-$ | Fp(°C) | C | H | N | Z | KMK X $10^{-6}$ Mol/Liter |
|---|---|---|---|---|---|---|---|---|
| 1. | 7-Hexadecylimidazolium-2,6-dihydroxy[4,5-d]pyrimidin | Cl · 1 $H_2O$ | 112 | 60,00 | 17,13 | 13,51 | | 0,50 |
| 2. | 7-Hexadecylimidazolium-2,6-diamino-[4,5-d]pyrimidin | Br · 1/2 $H_2O$ | 170(Zers.) | 55,17 | 8,93 | 18,41 | 17,49 | 1,30 |
| 3. | 3-Hexadecylbenzimidazolium | Cl · $H_2O$ | 100 | 72,53 | 10,80 | 7,35 | | 6,70 |
| 4. | 3-Hexadecyl-6-methyl-benzimidazolium | Cl · 2 $H_2O$ | 119(Zers.) | 69,81 | 14,13 | 7,09 | | 17,00 |
| 5. | 3-Dodecyl-6-methyl-benzimidazolium | Br · 1 $H_2O$ | 98 | 59,40 | 12,52 | 7,29 | | 7,30 |
| 6. | 3-Hexadecyl-5,6-dihydroxy-benzthiazolium | Cl · 2 $H_2O$ | 70 | 60,60 | 20,54 | 3,07 | 7,79 | 7,90 |

Tabelle 2

| Charakteristische Eigenschaften der hergestellten Substanzen | | | |
|---|---|---|---|
| N-Tensid | $Z^-$ | $\langle R_H \rangle$ | Å |
| 7-Hecadecyl-imidazolium-2,6-dihydroxy [4,5-d]-pyrimidin | $Cl^-$ | 110,0 | +/- 10,0 |
| | $Br^-$ | 350,0 | +/- 25,0 |
| | $H_2PO_4^-$ | 1.000,0 | +/- 100,0 |
| | Maleat | 1.000,0 | +/- 100,0 |
| 3-Hexadecylbenzimidazolium | $CL^-$ | 150,0 | +/- 10,0 |
| | Br | 1.000,0 | +/- 100,0 |
| | $H_2PO_4$ | 1.000,0 | +/- 100,0 |
| | Fumarat | 1.000,0 | +/- 100,0 |
| 3-Hexadecyl-6-methyl-benzimidazolium | $Cl^-$ | 350,0 | +/- 25,0 |
| | $Br^-$ | 400,0 | +/- 20,0 |
| | $H_2PO_4$ | 400,0 | +/- 20,0 |
| 3-Dodecyl-6-methylbenzimidazolium | $Cl^-$ | 100,0 | +/- 25,0 |
| | $Br^-$ | 1.000,0 | +/- 150,0 |
| | $H_2PO_4^-$ | 1.000,0 | +/- 150,0 |
| 3-Hexadecyl-5,6-dihydroxy-benzthiazolium | $Cl^-$ | 1.000,0 | +/- 25,0 |
| | $Br^-$ | 350,0 | +/- 25,0 |
| | $H_2PO_4^-$ | 1.000,0 | +/- 100,0 |
| | Fumarat | 1.000,0 | +/- 100,0 |
| 4-Dodecyl-5,6-dihydroxy-benzthiazolium | $Cl^-$ | 55,0 | +/- 10,0 |
| | $Br^-$ | 120,0 | +/- 11,0 |
| | $NO_3^-$ | 290,0 | +/- 20,0 |

Anwendungen:

Diese hier neu beschriebenen Verbindungen, haben überraschenderweise eine sehr kleine KMK im Bereich von $10^{-5}$ - $10^{-7}$ Mol/Liter, die weitgehend unabhängig ist von pH und Ionenstärke ($\leqq$ 0,1 M). Außerdem besitzen sie, da sie z.T. selbst Antimetabolite sind, biochemische bzw. pharmakodynamische Wirkung. Sie sind in der Lage die Zellmembranen von neoplastischen Geweben aufgrund ihrer "einwertigen" kationischen Natur zu penetrieren und sodann durch nicht bekannte Mechanismen nach Bildung der entsprechenden Nukleoside und Phosphorylierung in die Transkription als auch Translation inhibitorisch einzuwirken. Dies ist insbesondere von Bedeutung für infektiöse Prozesse bakterieller (Prophagen), oder vor allem viraler Natur.

Beachtenswert ist für eine spätere Anwendung dieser $N^+$-Tenside die Steuerung der kolloidchemischen "Aggregate" (Micellen) durch die Gegenionen $Z^-$, sowohl anorganischer als auch organischer Natur. Diese $N^+$-Tenside können im Gegensatz zu vielen anderen Amphiphilen, welche nicht wasserlöslich sind, aufgrund ihres hydrophoben Effektes "sheet-like" Doppelmembranstrukturen in Wasser oder wäßrigen Lösungen ausbilden. Meistens haben sie zylindrische Form, die allerdings sehr abhängig ist vom Gegenion: bei Anwesenheit von primärem Phosphat ($H_2PO_4^-$) als auch in Gegenwart von Glukonat oder Galaktoronat entstehen hoch orientierte micellare Faserstrukturen, die durch diese Anionen stabilisiert werden. So haben z.B. gelartige Präparationen, helikale Strukturen auf der Basis von micellaren Zylindern.

Die Erfindung offenbart weiterhin pharmazeutische Zubereitungen, enthaltend ein oder mehrere der N-alkylierten quartären stickstoffhaltigen Heterozyklen der allgemeinen Formel

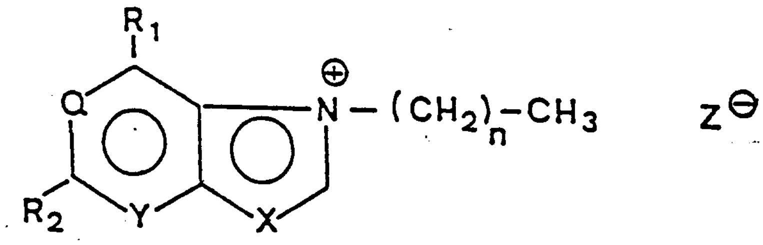

X = NH ; S
Y = N ; CH
Q = N ; C-$CH_3$ ; CH
$R_1$ = H ; OH ; F ; $NH_2$
$R_2$ = H ; OH ; $NH_2$
n = 8 bis 20 insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

und/oder eine oder mehrere der Verbindungen 3 und 4 der Seite 8, in die hydrophobe pharmazeutische Wirkstoffe bei pH 6 - 8 in polaren oder unpolaren Lösungsmitteln micellar oder vesikulär eingeschlossen sind.

Die Erfindung offenbart weiterhin pharmazeutische Zubereitungen, enthaltend ein oder mehrere der N-alkylierten quartären stickstoffhaltigen Heterozyklen der allgemeinen Formel

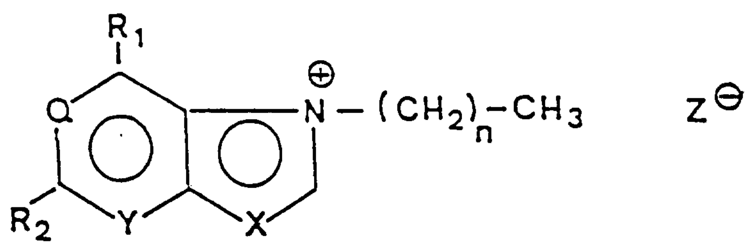

X = NH ; S
Y = N ; CH
Q = N ; C-$CH_3$ ; CH
$R_1$ = H ; OH ; F ; $NH_2$
$R_2$ = H ; OH ; $NH_2$
n = 8 bis 20 insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

und/oder eine oder mehrere der Verbindungen 3 und 4 der Seite 8 bei pH 6-8 in polaren Lösungsmitteln als eigenständige pharmazeutische Wirkstoffe, zusammen mit üblichen Zusatz- und/oder Hilfsstoffen und/oder weiteren pharmazeutischen Wirkstoffen.

Die Erfindung offenbart weiterhin pharmazeutische Zubereitungen zur Therapie von Magenulcera, die eine oder mehrere der Verbindungen 3 und 4 der Seite 8 als eigenständige pharmazeutische Wirkstoffe enthält.

Weiterhin offenbart die Erfindung die Verwendung von N-alkylierten quartären stickstoffhaltigen Heterozyklen der allgemeinen Formel

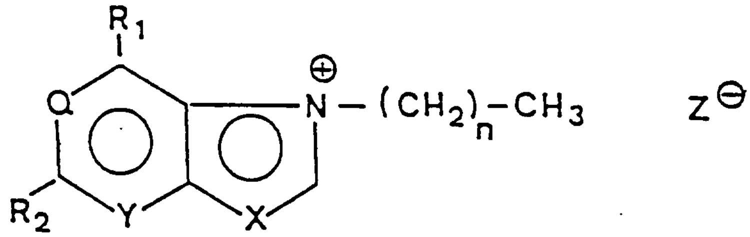

X = NH ; S
Y = N ; CH
Q = N ; C-$CH_3$ ; CH
$R_1$ = H ; OH ; F ; $NH_2$
$R_2$ = H ; OH ; NH⊂
n = 8 bis 20 insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

und der Verbindungen 3 und 4 der Seite 8 als spektroskopische Marker (Reporter-Gruppen) in immunologischen und klinisch-biochemischen Analyseverfahren sowie zu kolloid-chemischen Meßverfahren zur Bestimmung der KMK.

Micellen in wäßriger Lösung, sowohl nichtionische, kationische und anionische, sind in der Literatur in zahlreichen Veröffentlichungen beschrieben worden. (Mittal, K.L. (1977) Micellization, Solubilization and microemulsions, Plenum Press, New York. - Mittal, K.L. (1979), Solution Chemistry of Surfactants, Plenum Press, New York. - Menger, F.M. (1977). In Bioorganic Chemistry III. Macro- and Multicomponent Systems (E.E.Van Tanelen, Ed.), Academic Press, New York. - Menger, F.M. (1979a) Acc. Chem. Res. 12, 111-117. On the Structures of Micelles. - J.H.Fendler, E.J. Fendler (1975) Catalysis in micellar and macromolecular Systems, Academic Press.) Ihr Aufbau und ihre galenische, medizinische und technische Verwendung ist Gegenstand zahlreicher Untersuchungen. So ist die antiseptische Wirkung von Cetylpyridiniumchlorid, Benzethoniumchlorid und Benzalkoniumchlorid oder deren Bromide bekannt. Auch, daß sie in geringen Konzentrationen bakterizide Wirkung in vitro gegenüber einer großen Anzahl von grampositiven und gramnegativen Bakterien zeigen, wobei die gramnegativen wesentlich empfindlicher als die grampositiven reagieren. Auch sind bestimmte gramnegative Bakterien resistent gegenüber diesen quartären Ammoniumbasen, z.B. Pseud. cepalia, Mycobakt. tuberculosis.

Normalerweise haben kationische Micellen in wäßriger Phase zusätzlich in ihrem hydrophoben Kern, der weitgehend durch die aliphatische Kette und ihre Länge bestimmt wird, eine hydrophobe-hydrophile Grenzschicht (Sternlayer), die hydratisiert ist und z.T. die Gegenionen beherbergt. Die Größe dieser Grenzschicht liegt im allgemeinen zwischen 7-10 Å. Außerdem sind sie noch mit der Guy-Chapman-Layer von 10-20 Å umgeben, die nicht elektrostatisch gebundene Gegenionen, z.B. $Cl^-$, $Br^-$, $HSO_4^-$ und unstrukturiertes Wasser enthalten. Nur die Konzentrationen der Gegenionen als auch andere Ionen bewirken eine Senkung der kritischen Micellenbildungs-Konzentration (KMK) bei konstanter Temperatur, Druck und chemischen Potentials, wobei die Natur der Gegenionen die Form und Größe der Micellen in wässriger Phase bestimmen können. Dies bewirken allerdings nur die Fraktion von Gegenionen, welche im Stern-layer in der Nähe des quartären Stickstoffs sich befinden.

Die reinen, bislang bekannten kationischen quartären Ammoniumbasen - offiziell auch als Invertseifen bezeichnet - haben nur eine begrenzte und nicht spezifische antimikrobielle Wirkung (siehe z.B. W. Forth, D. Henschler, W. Rummel, Allgemeine und spezielle Pharmakologie und Toxikologie, 4. Auflage. B.I. Wissenschaftsverlag, 1983, S. 616). Daher sind ihre Einsetzbarkeit z.B. als Konservierungsmittel oder als Desinfiziens in den operativen Fächern der Medizin oder auf Infektionsstationen (Antiseptika) begrenzt, trotz ihrer geringen Toxizität. Domagk erkannte 1935 (siehe Wallhäußer, K.H.: Sterilisation, Desinfektion, Konservierung, Keimidentifizierung, Betriebshygiene. 2. Aufl., Thieme, Stuttgart, 1978), daß die quartären Ammoniumbasen nur dann bakterizid wirksam sind, wenn mindestens einer der Substituenten am Stickstoff aus einer linearen Alkylkette mit 8-18 Kohlenstoffatomen besteht, wobei die optimale Kettenlänge bei $C_{12}$-$C_{16}$ liegt. Die bekanntesten Vertreter dieser Stoffklasse sind die Benzalkonium-Salze (Chloride und Bromide). Darüber hinaus sind Hexadecylpyridinium-chlorid und Benzethonium-chlorid bekannt und haben medizinische und pharmazeutische Bedeutung erlangt. Die Wirkung dieser Invertseifen ist bekanntlich stark milieuabhängig. Durch Seifen z.B.

wird die Wirkung weitgehend aufgehoben, wie auch im sauren pH-Bereich. Auch Blut, Eiter, Stuhl sowie Schmutz führen gleichfalls zur Inaktivierung. Außerdem haben sie eine Eiweiß fällende Wirkung, die schon bei geringen Konzentrationen der $N^+$-Tenside einsetzt, d.h. im Bereich von 1-2 Gew.% von wäßrigen Lösungen. Bei Konzentration dieser bekannten Tenside, welche nur das 2-3-fache der kritischen KMK betragen, erfolgt zwar keine eiweißfällende Wirkung (Denaturierung), doch eine reversible Inaktivierung von Enzymsystemen und Stützproteinen durch Entfaltung der aktiven dreidimensionalen Struktur. ("loss of activity through unfolding")

Bekannt ist auch die antibakterielle und unspezifische Wirkung von quartären Ammoniumverbindungen und ihre oberflächenaktive Wirkung, von Dequalinium-acetat, Cetyldimethyl-ammonium-bromid (CTAB) und Hexadecyl-pyridiniumchlorid (CPCl), (siehe z.B. Goodman and Gilman's, The Pharmacological Basis of Therapeutics, EDS. A.G. Goodman, L.S. Goodman, Th.W. Rall, F. Murad, 1985, 7th Edition, Collier, MacMillan Publishing Company, N. Y., S. 971,; Merck Index, 1985). Die micellaren Eigenschaften dieser Verbindungen sind mit ihrer Oberflächenaktivität und antimikrobiellen Eigenschaften in Beziehung gesetzt worden (siehe Attwood, D, and Florence, A.T., Surfactant Systems, Chapman and Hall, London u. New York, 1983). Jedoch kann die unspezifische Oberflächenaktivität dieser quartären aliphatischen und aromatischen Ammoniumbasen nicht a priori als Voraussetzung für die antibakterielle, antifungale uund keratolytische Wirkung angesehen werden, da nichtionische Detergentien, z.B. Brij, Triton X 100, Lubrol etc. nicht reaktiv werden.

Organische quartäre Ammoniumbasen des Typs ($R_n$, $R_1$, $R_2$, $R_m$, $N^+$)$Y^-$, (HET≡$N^+$-$(CH_2)_x$-$CH_3$)$Y^-$ und $[H_3C)_3$.C-$CH_2$-$C(CH_2)_2$-$X_1$-[O-$(CH_2)_2]_2$-$N^+(CH_3)_2$-$CH_2$-$X_2$]$Y^-$ sind nur zum Teil bekannt, z.B. Hexadecyltrimethylammoniumchlorid und Bromid (Cetyltrimethilammonium-), Hexadecylpyridiniumchlorid bzw. Bromid (Cetylpyridiniumchlorid) und N,N'-Dimethyl-N- 2 2- 4-(1,1,3,3-tetrymethylbutyl)phenoxyethylphenylmethaniumchlorid (Benzethoniumchlorid, Methylbenzethoniumchlorid) und die Benzalkoniumchloride mit Alkylresten von $C_8H_{17}$ bis $C_{18}H_{37}$. Diese genannten $N^+$-Tenside haben alle eine kleine kritische Micellbildungskonstante (KMK) im Bereiche von $10^{-4}$-$10^{-5}$ Mol, je nach Milieubedingungen, wie z.b. Ionenstärke, Temperatur, Druck und Zusatz von organischen Lösungsmitteln bestimmter Dielektrizitätskonstanten. Der Einfluß eines Anions, $Y^-$, wie fraktionierte Bindungen, Zahl der Anionen an der Micelloberfläche (Sternlayer) und ihr Einfluß auf die geometrische Form der gesamten kationischen Micelle der oben genannten quartären organischen Ammoniumbasen ist bisher wenig untersucht. So auch die Form dieser o.g. Tenside in Gegenwart von Potenzierungsgemischen, wie Glyzerol, Dimethylsulfoxid, Ethanol, Propanol und ihre Stabilität gegnüber Temperatur und Aufnahmefähigkeit von hydrophoben (lipophilen) pharmazeutischen Wirkstoffen. Hier liegen keine quantifizierbare Untersuchungen auch der o.g. $N^+$-Tenside vor.

Tenside der allgemeinen Form (HET≡$N^+$-$(CH_2)_x$-$CH_3$)$Y^-$, wobei der Heterozyklus ein Benzimidazol-, oder Benzthiazol rest ist, sind bislang nicht beschrieben worden, sowie ihr micellares Verhalten in wäßrigen Lösungen in Gegenwart und Abwesenheit von Potenzierungsgemischen.

Der relativ breite und undifferenzierte Wirkungsmechanismus der bereits bekannten quartären organischen Ammoniumbasen und das damit bedingte Anwendungsgebiet als Antiseptika und ihre toxische Wirkung bei höheren therapeutischen Dosen, hat die Anwendung dieser organischen quartären Ammoniumbasen pharmazeutisch beschränkt. Auch ist für 1 Gew.%-ige oder höher wäßrige Lösungen, Cremen und Salben hypersensitive, allergische und topische Irritationen beobachtet worden, so daß ein gezielter therapeutischer Einsatz nur bedingt möglich ist.

Bekannt ist die bakterizide Wirkung von Chlorhexidin bei grampositiven und gramnegativen Bakterien, jedoch resistent gegen gramnegative Bazillen.

Pharmazeutische Präparationen, welche eine gezieltere Therapie mit micellar eingeschlossenen pharmazeutischen Wirkstoffen, z.B. antiviraler, antifungaler, antineoplastischer Natur, in therapeutisch wirksamen Dosen und in einer geeigneten pharmazeutischen Zubereitung (Galenik) ermöglichen, liegen nicht vor.

Ein großer Nachteil der bisher bekannten pharmazeutischen Zubereitungen von quartären organischen Ammoniumbasen, auch in Gegenwart von Potenzierungsgemischen, liegt in der Polydispersität der kolloidalen micellaren Lösungen. Je nach pharmazeutischer Zubereitungsform, pH-Wert, Ionenstärke, Gegenanion $Y^-$ und Temperatur liegen in einer pharmazeutischen Zubereitung bislang Micellen verschiedener Form und Größe sowie Stabilität und Aufnahmekapazität von pharmazeutischen Wirkstoffen vor.

Im weitesten Sinne versteht man unter Micellen durch Assoziation gebildete Aggregate von gelösten Molekülen. Im engeren, heute hauptsächlich verwendeten Sinn bezeichnet man als Micellen diejenigen Aggregate, die sich aus Tensid-Molekülen in wäßrigen Lösungen oberhalb einer bestimmten Temperatur (Krafft-Punkt) bzw. einer charakteristischen Konzentration bilden. Diese Konzentration nennt man kritische Micellbildungskonzentration (KMK; englisch: critical micellization concentration, cmc). Beim Überschreiten der KMK bleibt die Monomerenkonzentration praktisch konstant und die überschüssigen Tensid-Moleküle bilden Micellen. Diese können in verschiedener Gestalt (Kugeln, Stäbchen, Scheibchen) auftreten, abhängig von der chemischen Konstitution des Tensids sowohl von Temperatur, Konzentration oder Ionenstärke der Lösung. Die Micellen haben charakteristische Aggregationszahlen mit einer meist nur geringen Verteilungsbreite. Das Erreichen der KMK gibt sich durch sprunghafte Änderungen der Oberflächenspannung, (was man zur Messung der KMK ausnützt) des osmotischen Drucks, der elektrischen Leitfähigkeit und Viskosität zu erkennen.

Bei den Micellen handelt es sich um thermodynamische stabile Assoziationskolloide grenzflächenaktiver Stoffe, bei denen die hydrophoben Reste der Monomeren im Inneren der Aggregate liegen und durch hydrophobe Wechselwirkung (van-der-Waals-Kräfte) zusammengehalten werden; die hydrophilen Gruppen sind dem Wasser zugewandt und vermitteln durch Solvatation die Löslichkeit des Kolloids.

Weitere Informationen über Micellen finden sich in Römpps Chemielexikon, 8. Auflage, Franckh'sche Verlagsbuchhandlung Stuttgart, 1985, Seite 2600ff.

Die erfindungsgemäßen pharmazeutischen Zubereitungen enthalten den Wirkstoff in stabiler Form, wobei der Wirkstoff am Ort des pathologischen Geschehens relativ schnell und vollständig freigesetzt wird.

Die erfindungsgemäßen pharmazeutischen Zubereitungen sind gekennzeichnet, daß sie aufgebaut sind aus einer Micelle, bestehend aus einem kationischen Tensid mit einem einwertigen Anion und einem hydrophoben pharmazeutischen Wirkstoff, dispergiert in einem Lösungsmittel, dessen pH-Wert kleiner ≦ 7 ist, wobei die kritische Micellbildungskonzentration (KMK) im Bereich von $1{,}0 \,.\, 10^{-7}$ bis $1{,}5 \,.\, 10^{-4}$ Mol/Liter liegt.

Vorzugsweise ist diese pharmazeutische Zubereitung aufgebaut aus einer Micelle, bestehend aus einem kationischen Tensid mit einem einwertigen Anion in einer Menge von 0,01 bis 0,1 Gew.% bezogen auf die gesamte pharmazeutische Zubereitung, und einem hydrophoben pharmazeutischen Wirkstoff in einer Menge von 0,001 bis 0,5 Gew.% bezogen auf die gesamte pharmazeutische Zubereitung, dispergiert in einem Lösungsmittel, dessen pH-Wert ≦ 7,0 ist, in einer Menge von 99,40 bis 99,989 Gew.% bezogen auf die gesamte pharmazeutische Zubereitung, wobei die kritische Micellbildungskonzentration (KMK) im Bereich von $1{,}0 \,.\, 10^{-7}$ bis $1{,}5 \,.\, 10^{-4}$ Mol/Liter liegt.

Diese hier beschriebenen Micellen in wäßriger Phase haben bei einer hydrophoben Kettenlänge von 15-($CH_2$)-Gruppen einschließlich ihres quartären Stickstoffs im aromatischen Gebilde einen Durchmesser von ~ 50-100 Å (~ 50 - 100 x $10^{-10}$ µm), je nach Natur der Gegenionen.

<u>Weitere vorzugsweise Ausführungsformen der Erfindung:</u>

Während der Gesamtbereich der kritischen Micellbildungskonzentration (KMK) im Bereich von $1{,}0 \,.\, 10^{-7}$ bis $1{,}5 \,.\, 10^{-4}$ Mol/Liter liegt, liegt die KMK vorzugsweise im Bereich von 1,0 bis $8{,}5 \,.\, 10^{-7}$/Liter.

Vorzugsweise ist das kationische Tensid mit dem einwertigen Anion in einer Menge von 0,05 bis 0,1 Gew.%, bezogen auf die gesamte pharmazeutische Zubereitung, enthalten.

Besonders gute Ergebnisse werden erzielt, wenn das kationische Tensid mit dem einwertigen Anion in einer Menge von 0,08 - 0,1 Gew.%, bezogen auf die gesamte pharmazeutische Zubereitung, enthalten ist.

Vorzugsweise ist der hydrophobe pharmazeutische Wirkstoff in einer Menge von 0,06 - 0,05 Gew.%, bezogen auf die gesamte pharmazeutische Zubereitung, enthalten.

Besonders gute Ergebnisse werden erzielt, wenn der hydrophobe pharmazeutische Wirkstoff in einer Menge von 0,001 - 0,005 Gew.%, bezogen auf die gesamte pharmazeutische Zubereitung, enthalten ist.

Vorzugsweise Lösungsmittel sind Wasser oder Wasser + Glycerin oder Wasser + Glycerin + Ethanol.

Vorzugsweise ist das einwertige Anion ein monobasischer oder dibasischer Fettsäurerest.

Vorzugsweise ist das einwertige Anion Acetat, Propionat, Fumarat, Maleat, Succinat, Aspartat oder Glutamat.

Vorzugsweise ist das einwertige Anion ein Zuckerrest.

Vorzugsweise ist das einwertige Anion Glukonat, Galacturonat oder Alginat.

Vorzugsweise ist das einwertige Anion Chlorid, Bromid, Jodid oder Hydrogensulfat.

Vorzugsweise ist der hydrophobe pharmazeutische Wirkstoff ein antimikrobieller Wirkstoff oder ein antifungaler Wirkstoff oder ein antiproliferativer Wirkstoff oder ein antiviraler Wirkstoff.

Vorzugsweise ist der hydrophobe pharmazeutische Wirkstoff eine anorganische Verbindung der Elemente Zink oder Quecksilber oder Wolfram und/oder Antimon. Vorzugsweise ist dabei die anorganische Verbindung $Z_nSO_4$ oder $Z_nO$ oder $Hg(CN)_2$ oder $(NH_4)_{18}$ $(NaW_{21}$ $Sb_9O_{86})_{17}$ oder auch ein Alkali- oder Erdalkalisalz der Phosphonsäure $ROP(O)Me_2$ oder auch ein N-Phosphonoacetyl-1-aspartat.

Vorzugsweise ist der hydrophobe pharmazeutische Wirkstoff ein Antibiotikum oder ein antiviraler Wirkstoff oder ein antifungaler Wirkstoff oder ein antineoplastischer Wirkstoff.

Vorzugsweise ist das Lösungsmittel Wasser und/oder Ethanol und/oder Glycerin. Vorzugsweise ist das Lösungsmittel Wasser und/oder Ethanol und/oder Dimethylsulfoxid.

Während der pH-Wert des Lösungsmittels jedenfalls ≦ 7 sein muß, ist der vorzugsweise pH-Wert des Lösungsmittels = 5 bzw. in der Nähe von 5.

Die pharmazeutische Zubereitung kann erfindungsgemäß im wesentlichen dadurch hergestellt werden, daß zunächst in einem Reaktionsgefäß das Lösungsmittel vorgelegt wird, dann das kationische Tensid unter Rühren bei Zimmertemperatur zugegeben wird, dann zur entstandenen isotropen micellaren Lösung der hydrophobe pharmazeutische Wirkstoff unter Rühren bei Zimmertemperatur zugegeben wird und zu dessen vollständiger Lösung weitergerührt wird.

Besonders günstige Ergebniss werden mit den kationischen Tensiden erzielt, wenn die Alkylkette 15 C-Atome aufweist.

Diese geradkettigen $C_{15}$-Abkömmlinge der N-Tenside zeichnen sich insbesondere aus durch eine einfache chemische Herstellung. Außerdem haben sie überraschenderweise die niedrigste KMK (diese liegt ca. bei 2,5 . $10^{-7}$ Mol/Liter). Sie sind weiterhin durch $Y^-$ sehr leicht zu steuern (Form, Molekulargewichtsverteilung, Polydispersität). Außerdem sind sie variabel aufgrund ihrer Größe der Alkylkette und daher bezüglich Aufnahme der pharmazeutischen Wirkstoffe. Schließlich zeichnen sie sich durch leichte Kristallisierbarkeit aus.

Nicht bekannt ist der erfindungsgemäße Einfluß des zugehörigen Anions ($Y^-$) auf die Micellengröße und die Form der Micelle.

<u>Herstellungsverfahren für die pharmazeutische Zubereitung:</u>

<u>Generelles zur Herstellung der wässrigen Phase:</u>

Um vorzugsweise eine monodisperse, homogene und isotrope wäßrige Lösung der $N^+$-Tenside sowohl in Hinsicht auf Form (sphärisch, oval, langgestreckt) und Größe als auch auf Molekulargewichtsverteilung zu erreichen, müssen die angeführten Lösungen einschließlich ihrer eingeschlossenen hydrophoben pharmazeutischen Wirkstoffe

a. ultraschallbehandelt werden, z.B. bei 100 Watt, eine Minute, eventuell anschließend dann durch b.
b. durch Säulenchromatographie, z.B. auf einer Agarose A 0,5 m, Sepharose 2 B, Sephadex G 200, DEAE-Sepharose C1-6B bei pH 6,0 oder auf einem Ultragel AcA44 (pH 6.0 - 6.5) oder BiO-Gel 1,5 m bei pH ≦7,0 nachgereinigt; oder
c. auf einem linearen Dichtegradienten, z.B. von 1 - 30 Gew.% Subrose, in einer präparativen Ultrazentrifuge in einem SW-27-Rotor bei 25.000 UpM für 12 Stunden zentrifugiert werden. Bei Anwendung einer Zonal-Zentrifugation bei gleichem Gradienten (20°C) können bei 10,000 UpM große Mengen von homogenen Populationen von Micellen und Vesikeln zentrifugiert werden.
d. DEAE-Zellulose-Säulenchromatographie bei pH 5,0 - 6,5 (pH≦7), z.B. durch einen Phosphatgradienten (linear von 0,01M $KH_2PO_4$/0,01M $K_2HPO_4$, pH 6,5 bis zu 0,05M $KH_2PO_4$/0,05M $K_2HPO_4$ im totalen Elutions-Volumen von 1000 ml) gereinigt werden, bis die entsprechende, gewünschte Population von Micellen bzw. Vesikeln erhalten worden ist.

So ist es möglich, die gewünschten homogenen Populationen von Micellen oder Visikeln einschließlich ihrer eingeschlossenen pharmazeutischen Wirkstoffe, in Form von wiederholbaren konstanten Molekulargewichten und geometrischen Formen zu erhalten. Dies ermöglicht Monomere der Tenside von den Micellen als auch von nicht eingeschlossenen pharmazeutischen Wirkstoffen quantitativ zu trennen.

<u>Herstellung der homogenen, micellaren Lösung in wäßriger Phase:</u>

Die wäßrige Phase kann reines Wasser sein. In der Regel wird jedoch eine wäßrige Lösung eines Elektrolyten gewählt. Z.B. kann eine wäßrige Lösung aus NaCl oder $CaCl_2$ ($MgCl_2$) verwendet werden. Zusätzlich können aktive pharmazeutische Wirkstoffe von genannter Art eingeführt werden, die dann micellar gelöst werden auch eventuell unter Beschallung.

Die meisten Verfahren sind auf eine Einkapselung hydrophiler Wirkstoffe beschränkt. Mit der vorliegenden Erfindung ist es möglich, hydrophobe z.B. lipophile anorganische $(Hg)CN)_2$) und organische Wirkstoffe (Amphotericin B) micellar einzuschließen. Auch können hydrophile Anionen, die pharmazeutische Bedeutung haben, z.B. Salizylat, je nach Natur des N-Tensides (insbesondere der Formel II) an der externen Oberfläche der Micelle eingeschlossen werden.

Die Erfindung kann dazu verwendet werden, um entweder hydrophile oder lipophile Substanzen oder auch beide Substanzen einzuschließen. Im Falle von hydrophoben Wirkstoffen werden diese dann zusammen mit dem erfindungsgemäßen N-Tensid in einem Glycerin/Ethanol-Gemisch, bestehend aus 15 Gew.% Glycerin, 15 Gew.% Ethanol und 70 Gew.% Wasser oder 50 Gew.% Ethanol und 50 Gew.% Wasser, gelöst, eventuell geschüttelt bzw. ultraschall behandelt und anschließend auf die wäßrige Phase mit einem Gehalt von Glycerin/Ethanol von maximal 15 g Glycerin, 5 g Ethanol in 100 g Wasser verdünnt. Anschließende Gel-Permeationschromotographie oder präparative HPLC- können ungewünschtes Material entfernen und eine homogene, isotrope Lösung liefern. Während hydrophobe Substanzen vornehmlich über eine organische Phase (50 %) und anschließende Verdünnung (Wasser) hergestellt werden, werden hydrophile pharmazeutische Wirksubstanzen vorzugsweise in der wäßrigen Flüssigkeit eingesetzt, die zur Dispergierung der micellaren Lösung benutzt werden. Im Bedarfsfalle können jegliche nicht aufgenommene, aktive Wirkstoffe aus der Dispersion unter Anwendung bekannter Techniken, wie z.B. Dialysieren, Zentrifugieren, Gel-Permeationschromotographie entfernt werden.

Die Form und Größe, als auch der Hydratationsgrad der micellaren Lösungen der N-Tenside ist u.a. abhängig von $y^-$, weniger von der Struktur des Heterozyklus, wohl aber von der hydrophoben Kettenlänge $(CH_2)_x$. In diesem Falle

gibt die Form und Größe der Micelle die Konzentration des zu verkapselnden (micellar) Wirkstoffes an und gestaltet sich somit umgekehrt wie bei Liposomen.

Der Vorteil der Erfindung gegenüber der Verkapselung bei Liposomen liegt

1. in der Dichtigkeit dieser N-Tenside, welche den micellar gebundenen pharmazeutischen Wirkstoff aufgrund der vorne aufgeführten Kräfte nicht freilassen kann und
2. der Steuerung der Form und Größe der Micellen durch $y^-$, damit Steuerung der Aufnahmekapazität an hydrophoben bzw. hydrophilen Wirkstoffen, ohne großen, tiefgreifenden Einfluß des Heterozyklus auf die KMK.

Die erfolgte Bildung der kleinen und großen Micellen der N-Tenside in wäßriger Phase lassen sich anhand von physikalischen Meßmethoden nachweisen, z.B. mit gefriergetrockneten Proben ("freeze fracture") im Elektronenmikroskop oder durch Röntgenkleinwinkel-Streuung, durch dynamische Lichtstreuung, durch Kernresonanzspektroskopie ($^1H$, $^{13}C$ und $^{31}P$), als auch durch Transmissionselektronenmikroskopie.

Im Kernresonanzspektrum ergeben sich scharfe Signale mit schwacher Linienbreite, welche einen Hinweis auf die Bildung von Micellen mit einem Durchmesser kleiner als 600 Å liefert. Scharfe Signale bei $\delta$ ca. 0,89 ppm ($-CH_3$), $\delta$ ca. 1,28 ppm ($-CH_2-$) und $\delta$ ca. 3,23 ppm ($-N-(CH_3)_2$ sind z.B. für die Micellen der vorliegenden N-Tenside charakteristisch. Für eingeschlossene Wirkstoffe in diesen Micellen der N-Tenside ist ein Methylsignal bei $\delta$ ca. 0,87 - 0,89 ppm charakteristisch, das jedoch in einem Triplett aufgespalten ist und eine wesentlich geringere Linienbreite hat als das Methylsignal, das als Singlett vorkommt bei $\delta$ = 0,89 ppm, welches allerdings nur von der Micelle herrührt.

Diese wäßrigen Phasen, welche die erfindungsgemäß erhaltenen Micellen mit eingeschlossenen Wirkstoffen enthalten, sind Verabreichungssysteme, die sich gegebenenfalls nach Konzentrierung, z.B. durch Ultrafiltration, Ultrazentrifugation oder Lyophilisieren mit anschließendem Auflösen in einer wäßrigen Phase, für die orale (p.o.) oder topische Verabreichung eignen.

Bei oraler Verabreichung können die micellar gebundenen pharmazeutischen Wirkstoffe der N-Tenside der wäßrigen Phase mit pharmazeutisch unbedenklichen Verdünnungsmitteln oder Trägern oder mit üblichen Zusätzen, z.B. Farbstoffen oder Geschmackstoffen, vermischt und als Sirup oder in Form von Kaseln verabreicht werden.

So besteht eine homogene isotrope micellare wäßrige Lösung vorzugsweise aus einem erfindungsgemäßen N-Tensid mit einem antiviralen Wirkstoff, insbesondere mit $Hg(CN)_2$, oder $ZnSO_4$, ZnEDTA, Idoxuridin, 5-Ethyl-2'-desoxyuridin oder Trifluorthymidin, Amantadin, Rimantadin ($\alpha$-Methyladamantan) und Viderabin (9-$\beta$-Arabino ⟨1,4⟩-adenin) und Ribavirin (1-$\beta$-D-Ribofuranosyi-1,2,4-triazole-3-carboxamid) als auch mit 2,6-Di-amini-kuban 1,1':3,3'-Bis-cyclobutan oder einfach substituierten 2,6-di-amino-Verbindungen ($CF_3$,Cl,$OCH_3$) in Gegenwart oder Abwesenheit von Glycerin/Ethanol dispergiert (20°C; Ionenstärke <0,2 M).

Eine homogene isotrope micellare wäßrige Lösung besteht aus einem erfindungsgemäßen N-Tensid, vorzugsweise mit einem antifungalen Wirkstoff, vorzugsweise mt 5-Fluorcytosin, Clotrimazol, Econazol, Miconazol oder Oxyconazol (Z-Form) als auch mit Amphotericin B, Nystatin und ZnO.EDTA als anorganischer antifungaler Wirkstoff, sowie $Hg_2(CN)_4$ ($Hg(CN)_2$ liegt hier als Polymer vor, wobei das Dimere das zugrundeliegende Bauprinzip ist (in wäßriger Lösung) dispergiert.

Eine homogene, isotrope wäßrige Lösung besteht aus einem erfindungsgemäßen N-Tensid, vorzugsweise mit einem antineoplastischen Wirkstoff, insbesondere 5-Fluorcyanid, $Hg(CN)_2$.4 (Ascorbat oder Acetylacetonat), Azauridin, Cytarabin, Azaribin, 6-Merkaptopurin, Desoxycoformycin, Azathioprin, Thioguanin, Vinblastin, Vincristin, Daunorubicin, Doxorubicin in Gegenwart oder Abwesenheit von Glycerin/Ethanol dispergiert.

Eine homogene, isotrope wäßrige Lösung besteht aus einem erfindungsgemäßen N-Tensid, vorzugsweise mit Aminoglykoside wie z.B. Canamycin, Gentamycin, Neomycin etc. oder Tetrazyklinen, Chloramphenicol oder Erythromycin als bakteriostatische (grampositive) oder Clindamycin (gegen nicht sporenbildende anaerobe Bakterien) oder Rifampicin als bakteriziden, als auch Bacitracin, Tyrotricin und Polymycine in Gegenwart oder Abwesenheit von Glycerol/Ethanol dispergiert.

Die homogene Mischung kann auch anschließend in Gelen auf der Basis von Alginat, Hydrogelstrukturen wie z.B. Sephadex Agarose, Propyl-zellulose, Propyl-hydroxy-zellulose, in Gegenwart von DMSO, Glycerol dispergiert werden, wobei die pharmazeutischen Wirkstoffe micellar bei den gewünschten Konzentrationen enthalten sind.

Man dispergiert z.B. durch Schütteln, Rühren der wäßrigen Phase oder durch Ultraschallbehandlung, welche die zuvor hergestellte homogene isotrope Mischung enthält. Die Bildung der micellaren Strukturen mit den eingeschlossenen Wirkstoffen, pH $\leqq$ 7,0, 20°C, findet spontan, d.h. ohne große zusätzliche Energiezufuhr von außen und mit großer Geschwindigkeit statt. Die Konzentration an N-Tensid der Erfindung und Einschlußverbindung kann erhöht werden, wenn die KMK um mindestens das Zehnsache überschritten wird in der wäßrigen Phase bei konstantem chemischen Potential und Temperatur.

Die KMK ist eine variable Größe für die Menge der Monomeren der N-Tenside, welche man in einem bestimmten Volumen Wasser unter Verwendung von pH-Schwankungen $\leqq$ 7,0 lösen kann. Die KMK, die erfindungsgemäß nicht sehr abhängig ist von der Natur des Gegenions, welches nur die Form bestimmt, da weit oberhalb der KMK gearbeitet wird, kann durch elektrochemische Verfahrenn (Leitfähigkeit, Potentiometrie) durch Messung der Überführungszellen

im Zusammenhang mit den Gegenionen, der Oberflächenspannung, Dampfdruckerniedrigung, Gefrierpunkterniedrigung und osmotischer Druck, Messung der Dichte, des Brechungsindex, der elastischen und unelastischen Lichtstreuung (Diffusionskoeffizienten, Stokes' Radius) und der Viskosität, sowie durch Gelfiltration und Röntgenkleinwinkel-Streuungsmessungen bestimmt werden. Nanosekunden Fluoreszenz als auch die Messung der Fluoreszenspolarisation lassen zusätzlich Bestimmungen der Lage der eingeschlossenen pharmazeutischen Wirkstoffe durch die erfindungsgemäßen N-Tenside zu, z.B. durch ZnEDTA oder $Hg(CN)_2$ als Quencher und Amphotericin B als Verstärker. Positronium-Vernichtungs-Messungen an diesen beschriebenen micellaren Lösungen mit den eingeschlossenen Wirkstoffen lassen ebenfalls Aussagen über die Menge (Konzentration) des eingeschlossenen pharmazeutischen Wirkstoffes in Abhängigkeit von der Natur und Konzentration von $y^-$ zu.

Wäßrige Phasen mit einem pH-Wert >7,0 werden nach der Dispersion zentrifugiert. Die Neutralisation auf pH $\leqq$ 7,0 ist notwendig, um eine Zerstörung des Heterozyklus als auch des Wirkstoffes und/oder der Micellen unter basischen Bedingungen zu verhindern. Physiologisch gängige und verträgliche Säuren sind z.B. verdünnte wäßrige Mineralsäuren und Salzsäure, Schwefelsäure oder Phosphatsäure oder organische Säure, z.B. Niederalkansäuren wie Essigsäure oder Propionsäure. Meistens reagieren die wäßrigen Phasen der kationischen N-Tenside der Erfindung sauer bis neutral, können aber auch durch Sörensen-Puffer oder organische Inertpuffer, die z.B. HEPES, MOPS oder MES auf pH-Werte zwischen 3 und 7,0 genau eingestellt werden.

Herstellung der homogenen, micellaren Lösung in nichtwäßrigen Phasen:

Die Auswahl der betreffenden Lösungsmittel ist von der Löslichkeit des betreffenden pharmazeutischen Wirkstoffes darin abhängig. Geeignete Lösungsmittel sind z.B. Methylenchlorid, Chloroform, Alkohole, z.B. Methanol, Ethanol und Propanol, Niederalkancarbonsäureester, (Essigsäure-Ethylester), Ether oder Mischungen dieser Lösungsmittel. Nach Herstellen der micellaren Lösung und Zugabe des pharmazeutischen Wirkstoffes, gelöst im organischen Lösungsmittel, wird das organische Lösungsmittel entweder durch die vorne erwähnten Verfahren a. - d. oder durch Abblasen mit Inertgas, z.B. Helium oder Stickstoff, entfernt.

Pharmakodynamische Versuche :

Die Redeutung hochreaktiver Sauerstoffmoleküle (Superoxidradikale $O_2$, Peroxide $H_2O_2$, Hydroxylradikale $^{\cdot}OH$, Singulettsauerstoff $^1O_2$) im entzündlichen Prozess ist bekannt (s. z.B. McCord, J.M, K. Wong: Phagocytosis-produced free radicales: roles in cytotoxicity and inflammation. In: Oxygen Free Radicales and Tissue Damage, Excepter Medica, Amsterdam-Oxford-New York, 1979, 343-360; Allgemeine und spezielle Pharmakologie, Herg. W. Forth, D. Henschler, W. Rummel, Biowissenschaftlicher Verlag, 1983). Sie entstehen u.a. bei der Phacocytosen durch aktivierte Leukozyten (Monozyten, Makrophagen, polymorphkernige, neutrophile Granulozyten) und können zur Abtötung körperfremder Zellen, wie Bakterien, Bazillen u.a. auch bei bestimmten Viren, wenn das Immunsystem und die für IgG bzw. dem Komplementanteil $C_3$-spezifische Rezeptoren der Phagozyton funktionstüchtig sind, dienen. Die phagozytierenden Zellen selbst werden durch ein System, bestehend aus mehreren Enzymsystemen vor Schädigung durch diese besonders aktiven Formen des Sauerstoffs intrazellulär geschützt.

Es wurde nun gefunden, daß die quartären Ammoniumbasen der Erfindung, alle in der Lage sind, bei pH $\leq$ 7,0 diese Sauerstoffradikale gemäß dem nachfolgenden Reaktionsmechanismus zu eliminieren:

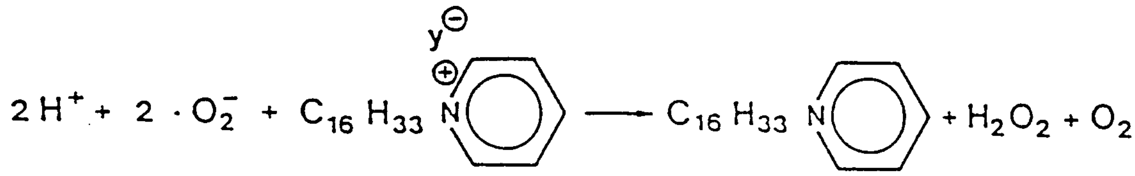

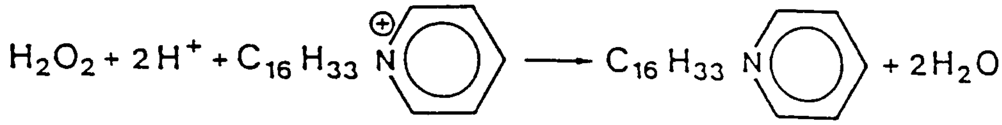

$$H_2O_2 + \cdot O_2^- \xrightarrow[pH \leq 6{,}0]{CP\ CL} 2\ OH^{\ominus} + O_2$$

$$\cdot O_2^- \xrightarrow{H_2O} e^{\ominus}_{H_2O} + O_2$$

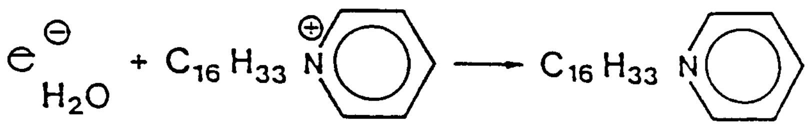

In allen Reaktionen, die im entzündlichen Gebiet zwischen pH 5,0 und 6,0 ablaufen, verlangen, was durch die verfahrensmässige Herstellung dieser Erfindung gewährleistet ist, einen pH-Bereich $\leq$ 7,0. Dadurch werden die gebildeten, agressiven Sauerstoffradikale gemäß der Reaktion 1-4 durch das N-Tensid,z.B. Cetylpyridiniumchlorid, als auch die entstehenden hydratisierten, kurzlebigen Elektronen, die durch Zusammenstoß $\cdot O_2^-$-Radikale mit $H_2O$ entstehen können, abgefangen. Dadurch haben die N-Tenside in dem pH-Bereich $\leq$ 7,0 erfindungsgemäß eine membranprotektive Wirkung, so daß es nicht zu Entzündungsreaktionen gemäß eines Prostaglandinmechanismus kommen kann. Die hohe Einfangrate $\cdot O_2^-$-Radikale bei diesen N-Tensiden von $k = 5 \times 10^{12} N^{-1} sec^{-1}$ und ihrer Abhängigkeit von der Ionenstärke, die allerdings durch Zusatz von Ethanol/Glycerol konstant gehalten werden kann, wird durch die elektrostatische Doppelschichtstruktur der quartären Ammoniumbasen erklärbar.

So werden erfindungsgemäß fehlgeleitete lytische Reaktionen, an denen aggresive Sauerstoffradikale beteiligt sind, als pathogene Mechanismen der entzündlichen Erkrankungen, hervorgerufen durch Mikroorganismen und Viren, verhindert. So werden auch u.a. die cytotoxische Wirkung der Folgeprodukte dieser agressiven $\cdot O_2^-$-Radikale durch die erfindungsgemäßen N-Tenside, wie am Beispiel von Cetylpyridiniumhalogenid gezeigt, verhindert und u.a. Depolymerisationen von Hyaluronsäuren, Proteoglykanen, Collagenfibrillen, Cytoskeletons usw. und auch bei mükosen und membranösen Geweben (äussere Oberflächen) verhindert.

Weiternhin wurde gemäß der beschriebenen verfahrensmässigen Herstellung gefunden, daß die erfindungsgemäßen Verbindungen die Infektion von menschlichen Zellen in vitro vermindert wird, so daß die erfindungsgemäß hergestellten micellaren Lösungen einen Schutz für die Zellen bzw. deren externer Oberfläche darstellen.

Es wurde weiter gefunden, daß dieser Schutz durch Inkorporation von $Hg(CN)_2$, ZnEDTA und/oder antiviralen, antifungalen und antibakteriellen Wirkstoffen verstärkt wird:
Es wurde gefunden, daß bei Inkubation von mit Influenzavirus, Untergruppe $A_2$, infizierte Monolayer-Zellkulturen von Vero-Zellen als auch bei Herpes simplex-Virus HSV I-III in vitro mehr als 60 % der Zellen vor der Infektion durch das betreffende Virus geschützt werden.

Es wurde weiter gefunden, daß der Effekt der Projektion durch die $N^+$-Tenside gemäß der Erfindung für Mono-layer Zellfunktionen in vitro durch die antiviralen Wirkstoffe nicht verstärkt wird, wohl aber werden die Hemmkonzentrationen der antiviralen Wirkstoffe von Cytarabin, Idoxuridin, Trifuorthymidin als auch Herpes simplex Virus Typ 1 oder Influenza Virus Typus $A_2$ infizierte Monolayer-Zellen um 30 % gesenkt gegenüber Applikationen, die keine quartäre Ammoniumbasen gemäß Formel I und II enthielten. Die Kombination von $N^+$-Tensid gemäß der Erfindung erwies sich somit als das wirksame Virustatikum in Kombination mit micellar gebundenen antiviralen Wirkstoffen.

Es wurde weiter gefunden, daß die $N^+$-Tenside gemäß der Erfindung die antifungale Wirkung in Kombination mit antifungalen Wirkstoffen wie Econazol, Clotrimazol, Miconazol verstärkt (≈ 35 %), da die $N^+$-Base bei geeignetem Gegenion in der Lage ist, Cholesterol aus der externen Membran des Pilzes oder Hyphen unter Bildung von gemischten Micellen ("mixed micelles") zu extrahieren und dann die antifungalen Wirkstoffe, die wieder gebunden sind, in das Zellinnere des Fungus injizieren kann.

Es wurde weiter gefunden, daß die antifungale Wirkung durch Amphotericin B und ein N-Tensid der Erfindung durch einen bislang nicht bekannten Mechanismus um das zehnfache verstärkt wird. Dies beinhaltet erfindungsgemäß, daß eine wesentlich geringere Wirkstoffkonzentration des pharmazeutischen Wirkstoffes ausreicht, um die gleichen therapeutischen Effekte zu erreichen.

Es wurde u.a. gefunden, daß die fungistatisce Wirkung durch micellaren Einbau von ZnEDTA und ZnO, insbesondere auch durch $Hg(CN)_2$ in die N-Tenside der Erfindung verstärkt wird, insbesondere bei Konzentrationen der anorganischen Wirkstoffe, wo sie selber noch nicht wirksam sind.

Es wurde gefunden, daß erfindungsgemäß die Micellen der N-Tenside in wäßriger Phase bei pH $\leqq$ 7,0 therapeutische Mengen von Benzoylperoxid, das schlecht wasserlöslich und alkohollöslich ist, micellar binden können. Bei örtlicher Anwendung lost die micellare Lösung ähnliche Schäleffekte an der Haut aus wie Tretinoin. Aufgrund seiner zusätzlichen sehr guten bakteriostatischen Eigenschaften, sowohl des Benzoylperoxides als auch des N-Tensides, ist erfindungsgemäß diese Kombination bei entzündlichen Akneformen besonders geeignet, z.B. bei Acne comedonica, Acne paculo-pustulosa und Acne conglobate.

Es wurde gefunden, daß die erfindungsgemäß hergestellten Micellen in wäßriger Phase der N-Tenside, in denen $Hg(CN)_2$ oder ZnO, $ZnSO_4$, ZnEDTA micellar eingeschlossen ist, in der Zellkultur irreversibel und virusspezifisch die Vermehrung von Herpes simplex Viren infolge Hemmung der viralen DNS-Polymerase, hemmen. Die nichtinfizierten Zellen bleiben weitgehend unbeeinflußt, so daß die in dieser Erfindung beschriebenen Verfahren, einschließlich der vorne genannten anorganischen Wirkstoffe zu einem risikolosen Therapeutikum führt. Die adstringierenden Eigenschaften von $Hg(CN)_2$, ZnO, $ZnSO_4$, ZnEDTA, spielen keine Rolle, da im hydrophoben Core der Micellen keine freien Ionen vorliegen, a) da z.B $Hg(CN)_2$ (richtiger $Hg_2(CN)_4$) undissoziiert ist, b) die anorganischen Wirksubstanzen durch ihre Lipophilie eingeschlossen sind und c) fast kein Wasser im hydrophoben Kern vorliegt.

Der kombinierte Effekt, die Bildung von gemischten Micellen ("mixed micelles") der N-Tenside gemäß der Erfindung mit der durch den Virus befallenen Membran <u>und</u> der Phospholipid-Doppelmembran des Virus selber und die anschließende antivirale Wirkung auf die Virus DNS-Polymerase durch die vorne genannten anorganischen und organischen Wirkstoffe wie der Nukleosid analoge, 5-Ethyl-2'-desoxy-uridin und Viderabin, konnte nachgewiesen werden.

Dieser Mechanismus konnte auch bei Rhino- und Influenza-Viren nachgewiesen werden. Ähnliche Wirkungen, jedoch bei geringeren Wirkstoffkonzentrationen wurden für 1,1':3,3'-Biscyclobutan und für 1,1':3,3' Amin substituierte Kubane gefunden.

Es wurde gefunden, daß die verstärkte antivirale Wirkung bei Phospholipid-Viren, Adeno-Viren und Herpes simplex I durch das N-Tensid und die micellar eingeschlossenen Wirkstoffe gemäß folgenden biochemischen Mechanismen ihre Wirkung synergistisch entfalten:

a) Bindung an DNS, RNS-bildende Enzymsysteme, Entfaltung dem Polypeptidkette durch das N-Tensid (Denaturierung) verstärkte.

b) "template"-Bindung, z.B. Daunomycin, Adriamycin

c) Bindung an Nukleosidsnaloga, z.B. das vorne erwähnte ara-CTP-C5'-triphosphat des Cytosin-arabinosids, Azathioprin;

d) Bindung von anorganischen Wirkstoffen, z.B. $ZnSO_4$, ZnO, $Hg(CN)_2$, Wolframsäure-antimonate, z.B. $(NH_4)_{18}(NaW_{21}Sb_9O_{86})_{17}$ und $K_{18}(KW_{21}Sb_9O_{86})_{17}$, sowie Hg-substituierte Kubane des vorne genannten Typus. Im Zusammenhang mit der antiviralen Wirkung der micellar eingeschlossenen antiviralen Wirkstoffe gemäß der verfahrensgemäßen Bearbeitung ist eine Reduktion der $ED_{50}$ um 20-25 % in vitro gegenüber dem reinen Wirkstoff zu verzeichnen, so daß die gleiche molekularbiologische Wirkung bei einer ca. 20 %igen Dosis durch den micellaren Effekt zu erzielen ist. Dies gilt insbesondere für micellar eingeschlossenes Rubaricin. Hexadecyl-benzothiazolium-chlorid DNA-Viren, Herpes-Viren sind bei diesen Beispielen am sensitivsten im Gegensatz zu Rimantadin + N-Tenside der Erfindung, welche primär in vitro gegen RNA-Viren wirksam sind.

Es wurde weiter gefunden, daß die Antitumoraktivität von Adenosin-Desaminase Inhibitoren, die micellar in einem erfindungsgemäße Tensid gelöst sind, z.B. Erythro-9-(2-hydroxy-3-nonyl)-Adenin, Deoxycoformycin, um das Zehnfache

verstärkt wird. Das gleiche konnte auch für Aspartat-Transcarbamylase Inhibitoren festgestellt werden; so wurde durch micellar eingeschlossenes N-(phospono-acetyl)-aspartat die Biosynthese von Pyrimidin durch Blockierung der Karbamylierung von Aspartat um das 20-fache verstärkt.

Außerdem wurde gefunden, daß sowohl micellar eingeschlossenes $Hg(CN)_2$, $ZnSO_4$, ZnO oder ZnEDTA, wie auch 5-Trifluor-methyl-2'-desoxyuridin, welches aus Trifluor-methyl-urazil in vitro entsteht, die Thymidin-Synthetase - ein Schlüsselenzym der DNS-Synthese - irreversibel hemmt.

Die Pyrimidin-Biosynthese wird durch Pyrazofurin, einem natürlich vorkommenden Antbiotikum, welches micellar eingeschlossen ist, um 20 % irreversibel gehemmt, bei gleichzeitiger Reduzierung der Zelltoxizität.

Es wurde weiterhin gefunden, daß die Diffusionsbarriere für Antibiotika, z.B. Tetrazykine, Aminoglykoside und auch für β-Lactamantibiotika (Penicillin), nach einer gewissen Zeit bei E. coli-Bakterien für die micellar eingeschlossenen Wirkstoffe herabgesetzt wird. Diese Diffusionsbarriere ist konzentrationsaßhängig für die vorne genannten Wirkstoffe, jedoch nicht für die erfindungsgemäß hergestellten N-Tenside. Hier handelt es sich um Faltungsprozesse an der äußeren Membran, primär um die Änderung der Struktur der Porine innerhalb der äusseren Membran von E. coli, so daß z.B. die anorganischen Wirkstoffe $Hg(CN)_2$, $ZnSO_4$, ZnEDTA in das Periplasma der äußeren Zellmembranen von gramnegativen Bakterien hinein diffundieren können.

Die Porine sind membranöse, wassergefüllte Poren, durch die hydrophile pharmazeutische Wirkstoffe in das Innere der Zelle diffundieren können Hydrophobe pharmazeutische Wirkstoffe können diese Porine nicht passieren. Die $N^+$-Tenside, insbesondere der allgemeinen Formel $HET{\equiv}N\text{-}(CH_2)_x\text{-}CH_3y^{\ominus}$ als auch Benzetoniumabkömmlinge könne diese wassergefüllten Poren passieren. Somit können micellar eingeschlossene pharmazeutische, hydrophobe (lipophile) Wirkstoffe, insbesondere anorganischer Natur, durch den hydrophilen, äußere Form der $N^+$-Tenside durch passive Diffusion ins Zellinnere gelangen.

Hier reagieren sie dann außerdem mit den Zellwand-synthetisierenden Enzymen, insbesondere mit $Hg(CN)_2$ bei Konzentrationen von 10 µg/ml, ZnEDTA bei c= 5 µg/ml.

Die Geschwindigkeit der Diffusion von micellar eingeschlossenen Wirkstoffen steigt mit steigendem hydrophoben Charakter, normalerweise ist dies genau umgekehrt, z.B. sinkt die Diffusionsgeschwindigkeit bei gramnegativen Bakterien mit steigendem hydrophoben Charakter. Weiterhin begünstigt eine positive Ladung die Diffusion und die Ausbildung von "mixed micelles" von diesen erfindungsgemäß herzustellenden N-Tensiden.

Die Gültigkeit dieser Befunde konnte durch Untersuchungen der Diffusions- und Auflösungsgeschwindigkeiten verschiedener radioaktiv ($C^{14}$) markierte N-Tenside an der Membran (Periplasma) konzentrationsabhängig nachgewiesen werden.

Es wurde außerdem in vitro gefunden, daß die Thymidilat-Synthetase (TS) (EC2.1.1.45) sowohl durch $Hg(CN)_2$ in wäßriger Lösung als auch in micellarer Lösung eines N-Tensides der Erfindung, in dem $Hg(CN)_2$ hydrophob gelöst ist, gehemmt wird. TS katalysiert die Umwandlung von dUMP und $CH_2$-$H_4$-Folat zu dTMP und $H_2$-Folat. Da dieses Enzym für die Synthese von dTMP essentiell ist, also für die DNS-Synthese schlechthin, stellt es somit auch ein Target für pharmazeutische Wirkstoffe gegen neoplastische Zellen dar. Es wurde nun gefunden, daß z.B. eine erfindungsgemäß hergestellte Lösung, die das $Hg(CN)_2$ hydrophob gebunden hält, zytostatische Aktivitäten gegenüber Leukämiezellen (L1210-Zellen) hat. So konnte u.a. gefunden werden, daß TS, dUMP und $Hg(CN)_2$ als anorganischer pharmazeutischer Wirkstoff einen ternären Komplex gemäß (A,B)

A

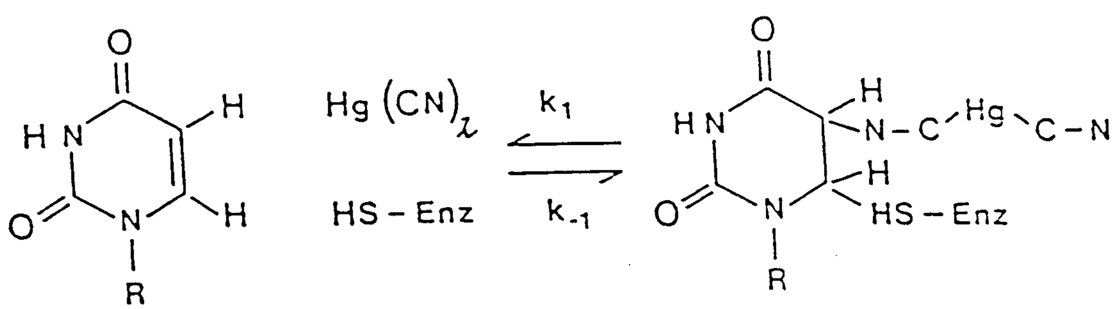

(d UMP)

R = DESOXy RiBOSE

B

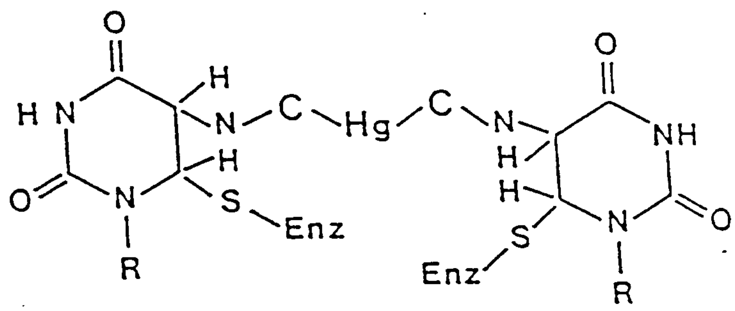

bilden, der durch Säulenchromatographie auf Sephadex G-25 und BiO-Gel P10 isoliert werden kann. D.h., daß micellare Lösungen von quartären Ammoniumbasen gemäß der Erfindung bei pH ≦ 7,0, welche $Hg(CN)_2$ hydrophob gebunden halten, sind daher therapeutisch bei langsam wachsenden Tumoren, wo andere Inhibitoren bei TS unwirksam sind und die beobachtete Cytotoxizität gegenüber den normalen Zellen von anderen Antimetabolite bei schnell wachsenden, proliferierenden Zellen kann daher verlangsamt werden.

Ribavirin, welches ein synthetisches 1,2,4-Triazolnukleosid ist, besitzt ein breites antivirales Spektrum für DNA- und RNA-Viren. Micellar eingeschlossenes Ribavirin durch kationische Tenside der Form $(Het{\equiv}N^+\text{-}(CH_2)_x\text{-}CH_3)Y^{\ominus}$ sehr schnell die Membranbarriere passiert, schneller als der pharmazeutische Wirkstoff selber. Auch die Unwandlung von Ribavirin zu Monophosphaten, Diphosphaten und Triphospaten durch die spezifischen zellulären Enzyme wird gesteigert, so daß die Hemmung der virusinduzierten Enzyme, welche notwendig sind für die virale Nukleinsäurebiosynthese, beschleunigt wird. Während Ribavirin alleine nur einen moderaten Effekt auf die zelluläre DNA-Synthese hat und zytotoxisch im Bereiche von 200-1000 µg/ml bei normalen Zellinien ist, sinkt die Zytotoxizität in Gegenwart von kationischen Micellen, wenn micellar eingeschlossen, auf 50 µg/ml (in vitro-Teste), gemessen gegen Herpes simplex (DNA-Virus) infizierten Zellen.

Amantadin (1-Adamantanamin-HCl) besitzt besondere pharmakodynamische Wirkung gegen Influenza-Viren (Klasse A). Die Replikation der meisten Influenza-A-Stämme werden in vitro zwischen 0,2 - 0,6 µg/ml gehemmt. Micellar eingeschlossenes Amantadin in kationische Micellen, insbesondere der Form $(Het{\equiv}N\text{-}(CH_2)_x\text{-}CH_3)Y^{\ominus}$ bewirken eine Reduzierung der Konzentration an pharmazeutischem Wirkstoff auf 0,05 µg/ml Amantadin gemessen nach Hayden et. al., (Plaque inhibition assay for drug suscentibility resting of influenca viruses. Antimicrob. Ag. Chermother. 1980, 17: 865-70, Grunert et al.; Sensitivity of influenza A/New Jersey 18/76/(Hswl-N1)-virus to amantadine HCl, J. Inf. Dis. 1977, 136, 297-300).

Bei einer Konzentration von 0,01 Gew.% von Amantadin bei Influenza-Virus Typ B entsprechend einer Konzentration von 0,5 µg/ml pharmazeutischen Wirkstoff in vitro.

Ein überraschender Effekt von micellar eingeschlossenem Amantadin in den beiden kationischen Tensiden der obigen Formeln wurde gefunden, das Influenza-Virus Typ A gegen Amantadin in vitro nicht resistent wird, während es bei Amantadin allein der Fall ist.

Rimantadin-HCl (α-Methyl-1-adamantanmethylamin-hydrochlorid) hat die gleichen pharmakodynamischen Wirkungen in vitro wie Amantadin, jedoch ist stärker wirksam bei gleicher Dosis. Auch hier wurde überraschenderweise gefunden, daß micellar eingeschlossenes Rimantadin in ein kationische Tensid, insbesondere der beiden obigen Formeln, der gleiche in vitro-Effekt gefunden wurde, wie beim reinen pharmazeutischen Wirkstoff, allerdings bei einer wesentlich geringeren Dosis von 0,05 µg/ml.

Sekundäreffekte

Die beobachteten Nebenwirkungen von Interferon $\alpha_1$, wie z.B. Kopfschmerzen, Lymphozytopenie, leichtes bis mittelschweres Krankheitsberinaen liegen bei den hier beschriebenen pharmazeutischen Zubereitungen, insbesondere gegen Influenza- und Rhinoviren, nicht vor bzw. wurden nicht beobachtet. Dies liegt vornehmlich daran, daß wesentlich geringere Einheiten/ml des Interferon α, induziert durch den anorganischen pharmazeutischen Wirkstoff, therapeutisch zur Anwendung kommen als bei einer Therapie mit Interferon α alleine. So wurden auch keine toxischen Effekte, z.B. gastrointestinale Störungen, Gewichtsverlust, Alopezia, periphere Krämpfe, Paraesthesien und Knochenmarksdeoressionen beobachtet, welche allerdings reversibel sind.

Die hämatologische Toxizität, welche im Falle von Interferon $\alpha_1$ dosisabhängig ist (Threshold-dosis $3x10^6$ Units/ml), liegt bei diesen pharmazeutischen Zubereitungen für Quecksilbercyanid, Rimantadin, Amantadin und Ribavirin nicht vor.

b) Die pharmazeutische Zubereitung, bestehend aus einem erfindungsgemäßen Wirkstoff in Gegenwart von micellar eingeschlossenem Quecksilbercyanid, bewirkt in der Zellkultur eine Interferonproduktion. Es handelt sich hier um eine Interferoninduktion durch freigesetztes Quecksilbercyanid, das örtlich in hohen Konzentrationen vorkommt und mit einem relativ hohem Molekulargewicht von 4500 durch Ausbildung von polymeren Strukturen. (Paradies, Oligomere Struktur von Quecksilbercyanid in Lösung, Vortrag Deutsch-Österreichische Chemische Gesellschaft, Innsbruck, Mai 1986). Somit gehört diese pharmazeutische Zubereitung zu den Stoffen definierter Interferoninduktoren von hohem Molekulargewicht wie z.B. synthetische doppelsträngige RNA:PolyI:PolyC als auch niedrigem Molekulargewicht wie z.B. 10-Carboxymethyl-9-acridanon. Trotz dieser Heterogenität der Interferonwirkung ist es antiviral wirksam. Diese Wirkung diente zum biologischen Nachweis dieser pharmazeutischen Zubereitung. Es kann gesagt werden, daß die Interferonbehandlung von Zellen in Zellkultur derartig verändert werden, daß eine nachfolgende Virusreplikation in diesen Zellen gehemmt wird. Dadurch unterscheiden sich Interferone in ihrem Wirkungsmechanismus grundsätzlich von Virustatika, die den Stoffwechsel der Viren selbst hemmen. Da Interferone auf die Zellen wirken, ist es nicht verwunderlich, daß sie auch andere Wirkungen auf die Zellen ausüben können. Dies gilt nicht nur für Zellkulturen sondern hat auch Auswirkungen für den Gesamtorganismus. Es ist bekannt aus vielfältigen Untersuchungen, daß Interferon eine Vielzahl von Viren in ihrer Vermehrung hemmt. Das Ausmaß der Hemmung ist abhängig vom jeweiligen Virussystem. Somit scheint die Vermehrung fast aller Viren durch Interferon-Behandlung der Zelle hemmbar zu sein. Es gibt offensichtlich verschiedene Mechanismen, mittels deren Interferone wirksam sein können. So wird z.B. die Vermehrung von Retroviren auf die Bildung des "budding" d.h. des Ausschleusens neu gebildeter Virionen beeinflußt. Ein ähnlicher Mechanisnus scheint auch für die pharmazeutische Zubereitung mit micellar eingeschlossenem $Hg(CN)_2$ zuzutreffen.

Die erfindungsgemäße pharmazeutische Zubereitung, speziell micellar eingeschlossenes Quecksilbercyanid in 7-Hexadecylimidazolium-2,6-diamino-[4,5-d]-pyrimidin-chlorid ließen die Interferon bedingte Hemmung bei verschiedenen lytischen RNA-Viren beobachten. Die Inhibition erfolgt auf der Ebene der Regulation der viralen Proteine. Sie wird hervorgerufen durch Induktion bestimmter zellulärer Enzyme. Bei näherer Untersuchung wurde gefunden, daß die Enzyme der 2',5'-Oligo-adenylat-Synthetase, der 2,5-A-abhängigen Endoribonuklease und die dsRNA-abhängige Proteinkinase hemmen. Für die beiden ersteren konnte durch Korrelationsstudien und durch Charakterisierung von Zellmutanten eine Rolle in der antiviralen Aktivität gegen lytische RNS-Viren durch micellar gebundenes $Hg(CN)_2$ nachgewiesen werden.

In diesen experimentell nachgewiesenen Effekten konnte auch ein antiproliferativer Effekt dieser pharmazeutischen Zubereitung auf die Interferone in vielfältiger Weise auf die Membranen und das Zytoskelett von Zellen nachgewiesen werden. So beeinflussen sie weiterhin die Expressionen einer Reihe wichtiger Gene, z.B. die der Haupthistokompatibilitätslokus, die sogenannten Transplantationsantigene. Somit zeichnen sich auch immunregulatorische Wirkungen ab (Interleukin-1-Synthese). Dadurch ergeben sich therapeutisch und pharmakodynamisch folgende Aspekte: Die Induzierung der Interferone durch diese pharmazeutische Zubereitung führt zu verstärkter Expression der Zelloberflächenproteine, die die wichtigste Rolle bei der Immunantwort spielen. Es sind dies die sogenannten Transplantationsantigene. Weiter ist anzuführen, daß mindestens zwei wichtige zelluläre Komponenten der körpereigenen Abwehr aktiviert werden, nämlich die Makrophagen und die natürlichen Killerzellen. Außerdem, was vor allem das Interferon $\gamma$ betrifft, scheinen auch die Funktionen vom B-Lymphozyten maßgeblich durch diese pharmazeutische Zubereitung beeinflußt zu werden.

Somit ergibt sich für die erfindungsgemäße pharmazeutische Zubereitung, insbesondere bestehend aus micellar eingeschlossenem Quecksilbercyanid und 7-Hexadecylimidazolium-2,6-diamino-[4,5-d]-pyrimidinchlorid oder auch -bromid, eine Induktion, wenn auch keine spezifische, von Interferon. Es kann kein Zweifel daran bestehen, daß Interferone nicht nur in vitro sondern auch in vivo nicht nur antiviral sondern auch immunregulatorisch wirksam sind. Obwohl der direkte antivirale Effekt auch in vivo bedeutsam sein kann, spielen im Organismus bei der Interferon-Wirkung, wie sie oben aufgezeichnet worden sind, weitere indirekte Mechanismen eine Rolle, z.B. die Aktivierung von Makrophagen speziell bei Influenza-Virus A und B. Die Tatsache, daß Interferon $\gamma$ Makrophagen aktivieren kann, scheint auch im Hinblick auf bakterielle und parasitäre Infektionen wichtig zu sein, da bei deren Abwehr Makrophagen eine entscheidende Rolle spielen.

<u>Posologie und therapeutische Indikationen</u>:

Die therapeutischen Indikationen sowie die Dosis ergeben sich durch die micellar eingeschlossenen Konzentrationen der pharmazeutischen Wirkstoffe:

- So bestehen Indikationen für aufkommende und bestehende Erkältungskrankheiten, die vornehmlich durch Influenza- und Rhinoviren verursacht werden;
- katarrhalische Entzündungen viruider Genese;
- Hautinfektionen und infektiöse Dermatosen;
- hartnäckige, antiseptische Wundbehandlung;
- Dermatomykosen;

- Mykosen;
- Prophylaxe und Therapie bakteriell bedingter Hautläsionen wie z.B. Pyodermie, Otitis media;
- mikrobielle und sekundär infizierte Ekzeme;
- Überempfindlichkeit gegen Makrolid-Antibiotika;
- Akne, insbesondere entzündliche Formen mit Papeln und Pusteln;
- bakterielle Infektionen und Sekundärinfektionen der Haut, sofern sie durch grampositive und/oder gramnegative meklocyclinsensible Keime hervorgeruzen werden;
- Akne vulgaris;
- Verhütung von Nabelinfektionen;
- chirurgische und traumatische Wunden;
- lokaler Schutz vor Infektionen und mit Antibiotika empfindlichen Keimen infizierte Wunden;
- Furunkel, Karbunkel, Abszess;
- Dermatomykosen, verursacht durch Dermatophyten, Hefen, Schimmelpilze und anderen Pilzen, Pityriasis versicolor,
- Erythrasma durch Cornebakterien;
- Candidosen der Haut und Schleimhäute
- Herpes simples I-III, Herpes-Keratitis
- solare und senile Keratosen, prämaligne Veränderungen und oberflächliche Basaliome, auch in strahlengeschädigter Haut;
- Plattenepitelkarzinome der haut und Mukosa im Kopf- und Halsbereich; dermatologische Malignome;

Je nach Indikationsstellung und pharmazeutischem Wirkstoff richtet sich die spezielle Dosis. Da die Erhaltungs- und Anfangsdosis der hier beschriebenen pharmazeutischen Wirkstoffe bekannt sind, wird je nach Applikationsart und galenischer Zubereitung z. B. Cremen, Zäpfchen, Tropfen, Tabletten, Kapseln und liposomartige Verkapselungen nur 50 % der normalen therapeutischen Gesamtdosis benötigt, je nach Konzentration des micellar eingeschlossenen pharmazeutischen Wirkstoffes.

<u>Schilderung der Dosisreduzierung aufgrund des potenzierenden (synergistischen) Effektes</u>:

Micellen in wässriger Lösung, auch mit eingeschlossenen lipophilen Wirkstoffen, stehen in dynamischen Gleichgewicht mit ihren monomeren Tensiden, d.h. die Micellen ändern Form, Größe und Hydratation. U.a. verlassen monomere kationische Tenside eine einzelne Micelle, um sich an einer anderen Micelle in wässriger Lösung wieder einzugliedern, so daß - auch wenn die Konzentration des Tensides weit über der KMK liegt - immer eine gewisse Konzentration von fluktuierenden Monomeren besteht. Durch Zugabe des Potenzierungsgemisches wird diese Dynamik dahingehend gestört, daß

1.) bei konstanter Temperatur und chemischen Potentials die Form, Größe und monodisperse Homogenität der isotropen Lösung erhalten bleibt, somit tritt auch kein Verlust an micellar eingeschlossenem lipophilen (hydrophoben) pharmazeutischen Wirkstoff ein.

2.) Die Konzentration an Monomeren, welche destabilisierend auf die geometrische Form der Micelle wirkt, wird zugunsten eines Einbaues in die"vollständigen" Micelle in isotroper Lösung eingeschränkt. Dies bewirkt, daß das System einschließlich der micellar eingeschlossenen hydrophoben pharmazeutischen Wirkstoffen "leckt". Dies wird vornehmlich dadurch verhindert, daß das Potenzierungsgemisch, insbesondere Glycerin und Dimethylsulfoxid, die Wasserstruktur an der externen Oberfläche der Micelle so einfrieren ("Tridymit-Struktur"), daß sie eisartige Strukturen annehmen und die Wassermoleküle sehr unbeweglich werden.

3.) Die pharmazeutische Zubereitung wirkt aufgrund des potenzierenden Effektes durch Glycerin, wie z.B. in vitro gezeigt werden konnte, weniger zytotoxisch, d.h. es schädigt vornehmlich die beschädigte (infizierte) Zelle,weniger die gesunde Zelle im Zellverband.

Die Erfindung weist insbesondere folgende Vorteile auf:
Die in dieser Erfindung hergestellten N-Tenside, einschließlich ihrer verfahrensgemäßen Einschließung der Wirkstoffe, bedingt eine erhebliche, z.T. bis zu 80%ige Reduzierung der Toxität der anorganischen Wirkstoffe, z.B. bei $Hg(CN)_2$, (auch $HgCl_2$, $Hg_2Cl_2$, $HG(NH_2)Cl$ [Präzipitat], ZnEDTA) und Zn Salzen im allgemeinen, als auch bei nephrotoxischen, ototoxischen Antibiotika, insbesondere bei Polymixinen, Erythromycin, Gentamycin, Tetrazyclin, von ca. 30 % da

1.) die Micellen, als auch ihre eingeschlossenen Wirkstoffe, nicht - wegen ihrer Größe - resorbiert werden,

2.) die micellar eingeschlossenen Wirkstoffe sich nur am Ort - meistens topisch - entfalten, so daß geringe Konzentrationen an Wirkstoff ausreichen, da zusätzlich noch der synergistische Effekt des N-Tensides besteht.

Auch stabilisiert z.B. Hexadecyl-benzthiazolium-sulfat das Atropin durch Einbeziehung der hydrophoben Molekülregionen des Atropins in den micellaren Kern.

Dieser Inhalt des Patentanspruches erstreckt sich auch auf die antiphlogistischen Eigenschaften der hier beschriebenen quartären organischen Ammoniumbasen. Das Gegenion $Y^-$ nimmt verfahrensbedingt Einfluß auf die Größe, Form und micellare Stabilität, kann aber auch selber ein pharmazeutischer Wirkstoff sein, so daß eine Arzneimittelwirkung pharmakodynamisch verstärkt werden kann. Die hier beschriebenen Tenside haben den großen Vorteil, daß sie neben intrinsischen pharmakodynamischen Eigenschaften milieuabhängig und darüber hinaus im sauren pH-Bereich stabil sind. Die verfahrensmäßigen erhältlichen Micellen als pharmazeutische Zubereitung mit eingeschlossenen lipophilen (hydrophoben) pharmazeutischen Wirkstoffen wirken als Träger für antimikrobielle, antivirale, keratolytische, antifungale und antineoplastische Wirkstoffe, können aber u.a. selber antimikrobielle, antifungal und antiviral und antiphlogistisch (topisch) wirksam sein.

Insbesondere beschreibt die vorliegende Erfindung eine pharmazeutische Zubereitung zur Herstellung von micellaren gelösten hydrophoben anorganischen Wirkstoffen wie Quecksilber-II-Cyanid, Zink, Wolfram und Antimon-Verbindungen sowie Salze der Phosphonsäure. Es wurde gefunden, daß diese anorganischen Verbindungen sowohl antivirale als auch antineoplastische Wirkungen haben.

Insbesondere bezieht sich die Erfindung auf ein verbessertes Herstellungsverfahren zur Erzeugung von multilamellaren Lipidbläschen auf der Basis von kationischen Tensiden, die dazu benutzt werden können, insbesondere lipophile (hydrophobe) pharmazeutische Wirkstoffe einzukapseln.

Die meisten bisher bekannten Verfahren leiden entweder an einer zu geringen Einkapselwirkung oder an einer Begrenzung der Materialtypen, welche eingeschlossen werden sollen, oder auch an beiden. So sind bekanntermaßen die meisten dieser Prozesse auf den Einschluß hydrophiler Materialien und pharmazeutischer Wirkstoffe beschränkt und können nicht wirkungsvoll den Einschluß von lipophilen pharmazeutischen Wirkstoffen vornehmen. Im Gegensatz dazu sind alle derzeit verfügbaren Verfahren mit Ausnahme das von Banghan et al. (Biochim.Biophys.Acta 443:629-634, 1976) nur für die Einkapselung biologisch aktiver Substanzen in oligo-multilamellaren oder unilamellaren Liposomen geeignet.

Ein besonderer Vorteil dieser pharmazeutischen Zubereitung auf der Basis vesikulärer Strukturen von $N^+$-Tensiden ist die hydrophobe Einkapselung von pharmazeutischen Wirkstoffen. Eine besonders vorteilhafte Folge der durch Ultraschallbehandlung und Gegenionen hergestellten großkalibrigen Vesikel ist darin zu sehen, daß die Gefahr des Austritts des pharmazeutischen Wirkstoffes aus der Bläschenhaut des Ansatzes reduziert oder eliminiert wird. Deshalb kann diese Form des quartären $N^+$-Tenside auf der Basis von sechsgliedrigen Heterozyklen, insbesondere für das Einkapseln hydrophober pharmazeutischer Wirkstoffe herangezogen werden, die dazu verwendet werden können, um lokale z.B. örtlich begrenzte statt systemische Wirkungen zu erzielen.

Während die meisten der bekannten Verfahren auf die Einkapselung hydrophiler Wirkstoffe beschränkt sind, kann mit dieser Erfindung die Einkapselung von hydrophoben pharmazeutischen Wirkstoffen vorgenommen werden. Versuche haben gezeigt, daß sogar anorganische lipophile pharmazeutische Wirkstoffe wie z.B. Quecksilber-II-Cyanid mit hoher Wirksamkeit eingeschlossen werden können und ihre pharmakodynamische Wirkung durch das Potenzierungsgemisch noch verstärkt werden kann.

Dieser Nachteil wird durch die neuen N-Tenside der Erfindung wie auch die Vesikel auf der Basis von $N^+$-Tensiden entweder durch micellaren Einschluß der pharmazeutischen Wirkstoffe oder durch kovalente Verknüpfung der Wirkstoffe mit dem $N^+$-Tensid unter Beibehaltung der äußeren morphologischen Form der gesamten Micelle aufgehoben.

Bekannt ist die bakterizide Wirkung von Chlorhexidin bei grampositiven und gramnegativen Bakterien, jedoch resistent gegen gramnegative Bazillen. Gefunden wurde, daß micellare Lösungen von quartären Ammoniumbasen gemäß der Erfindung die 2-4 Gew.% Chlorhexidin hydrophob im micellaren Kern gebunden halten, die Resistenz gegen gramnegative Bazillen aufgehoben und ihre therapeutische Wirksamkeit im Verhältnis zum Chlorhexidin alleine verstärken. Die beobachteten Nebenwirkungen von Chlorhexidin wie Kontaktdermatiden, Hauteffekte topischer Art, Photosensibilität der Haut, finden bei verfahrensgemäßer Herstellung der micellaren Lösungen der N-Tenside der Erfindung nicht statt.

Es ist Gegenstand der vorliegenden Erfindung, die eingangs erwähnte Heterogenität von Form und Größe der Micellen auch in Gegenwart von Potenzierungsgemischen abzustellen. Es wird somit gewährleistet, daß eine monodisperse Form von kationischen organischen Ammoniumbasen auch in Gegenwart von pharmazeutischen Wirkstoffen und Potenzierungsgemischen bei der Herstellung erreicht wird.

Diese erfindungsgemäßen quartären organischen Ammoniumbasen beseitigen die oben geschilderten Nachteile der bislang herkömmlichen Invertseifen. So besteht außerdem großes Interesse sowohl an der therapeutischen Verwendung von quartären Ammoniumbasen, die sowohl als pharmazeutischer Wirkstoff fungieren und als Träger von Wirkstoffen unterschiedlichster Art, z.B. antimikrobieller, antiviraler, antifungaler oder antineoplastischer Natur, micellar aufnehmen können. Sie sollen daher die eingangs genannten, vor allem milieu-bedingten Nachteile nicht besitzen.

Die erfindungsgemäßen, mit pharmazeutisch kovalent verbundenen Wirkstoffe, auf der Basis von quartären Ammoniumbasen, haben den Vorteil

1. daß diese maskierten Antimetabolite aus der Pyrimidin- bzw. Purin-Reihe, keine intramoleculare Wechselwirkungen anionischer bzw. kationischer Natur eingehen. Sie sind neutral geladen (z.B.kein Nukleotid-dianion durch das Phosphat) und können daher ungehindert in die pro- bzw. eukaryontische Zelle diffundieren, so daß hohe intrazelluläre Antimetabolit- (z.B. 5'-Nukleotid) Konzentrationen erreicht werden;

2. daß die pharmazeutischen Wirkstoffe durch N-C-Hydrolyse mittels der vorhandenen Enzymsysteme der germinalen bzw. eukaryontischen Zellen, erst am Target oder auch topisch freigesetzt werden;

3. durch die Erhöhung der Hydrophobizität der Alkyl- bzw. Aryl-Kette bzw. Restes am $N^+$-Tensid wird die Membran-Permeabilität erhöht, so daß die pharmazeutischen Wirkstoffe quantitativ passiv in das Zytosol übertreten können. Im Gegensatz zu Di-Anionen oder Kationen, welche die Membran unter physiologischen pH-Bedingungen und Ionenstärken schwer passieren können, ist dies bei den verfahrensgemäßen $N^+$-Tensiden ungehindert;

4. die hohe Hydrophobizität bedingt auch einen hohen Verteilungskoeffizient im System $CHCl_3$-$H_2O$ bei pH 8,0;

5. durch die konzentrierte Aufnahme von hydrophoben bzw. hydrophilen pharmazeutischen Wirkstoffen wird zusätzlich zu den kovalent verankerten und die Wirkstoffkonzentration nach Penetration durch die germinale Membran, fungale Zellwand (Hemmung der Chitinsynthetase) oder virale Phospholipid-Doppelmembran durch einen Konzentrationsgradienten (extrazellulär - intrazellulär) erhöht. Dadurch resultiert eine geringe Anflutungszeit.

Im Gegensatz zu Liposomen als Träger von pharmazeutischen Wirkstoffen, wie sie z.B. in der US-Patentschrift 3,993,754 oder in der europäischen Patentschrift 0,102,324 genannt sind, haben diese hier erfindungsgemäß beschriebenen Micellen von quartären Ammoniumbasen die Vorteile,

1. daß sie wasserunlösliche Wirkstoffe micellar im sogenannten "liquid core" aufnehmen können, und dadurch sowohl topisch als auch enteral durch Öffnen der Micelle diese wasserunlöslichen Wirkstoffe kontrolliert freisetzen können, z.B. Rimantadin, Amantadin, Tromantadin, die bei Influenza-Viren bzw. Herpes simplex-Viren sowohl der Haut als auch des Auges wirksam sind.

2. wasserlösliche Wirkstoffe können sowohl im Sternlayer als auch micellar gelöst werden, wenn sie selber hydrophobe Bereiche haben, wie z.B. Polyen-Verbindungen, Tetrazykline, Aminoglykoside und aromatische Antimetabolite, Z.B. Trifluorthymidin, Viderabin, Cytarabin, 5-Jod und 5-Fluor-desoxyuridin, 5-Ethyl-2'-desoxyuridin, Erythromycin und Nalidixinsäure.

3. Die hier erfindungsgemäß beschriebenen kationischen N-Tenside haben zwei spezifische Bindungsstellen mit hohen Bindungskonstanten ($K_B$ = 10-15µM) und großer Bindungskapazität (Kapazität 100 µg/Micelle): die eine ist bedingt durch die hydrophoben Wechselwirkungen zwischen dem "liquid core" der Micelle und dem hydrophoben Bereich des Wirkstoffs ( G=15-20kcal/Mol) als auch die $\pi$-$\pi$-Wechselwirkungen der hier beschriebenen Wirkstoffe zwischen den N-Heterozyklen der N-Tenside und den pharmazeutischen Wirkstoffen, die zweite Bindungsstelle ist unspezifischer Art und ist an der Grenzfläche zwischen Stern-layer und hydrophobem Kern lokalisiert. Die Bindungskonstante liegt im Bereich von $K_B$ = 20 mM, die Bindungskapazität 100-200 µg/Micelle. Die unspezifische Bindungsstellen liegen fast ausschließlich im Guy-Chapman-Layer. Im Gegensatz zu Liposomen, wo die Zahl der unspezifischen Bindungsstellen wesentlich höher ist, kann die Zahl der unspezifischen Bindungsstellen durch Zugabe von Ethanol/Glycerol ausgeschaltet werden, da die Kräfte, welche in dem Guy-Chapman-Layer wirken, durch Konzentrationen von Ethanol, Glycerin bis zu 30-35 Gew.% ausgeschaltet werden, ohne die Bindungskapazität, -stärke der hydrophoben Kräfte bzw. im Stern-layer zu beeinflussen (nur Polarität und Konfiguration).

4. Die hier beschriebene Erfindung hat den Vorteil, daß die micellar eingeschlossenen pharmazeutischen Wirkstoffe nicht wieder den micellaren Verband verlassen, wie z.B. bei Liposomen, die nach den bisher bekannten Verfahren "lecken". Die "Dichtigkeit" ("sealing") der vorliegenden Erfindung von micellar eingeschlossenen Wirkstoffen ist z.B. am Beispiel von micellar gebundenen radioaktiv markiertem Trifluorthymidin, Cytarabin und Idoxuridin nachzuweisen. So wurde u.a. gefunden, daß Idoxuridin erst nach 200 Tagen 5 Gew.% seiner ursprünglichen micellar eingeschlossenen Konzentration (2000µg) im Falle von Hexadecyl-pyridinium- oder Benzethonium-chlorid Micellen verliert. Die entsprechenden Werte für radioaktiv markiertes Trifluorthymidin und Cytarabin liegen bei 210 und 300 Tagen (20°).

5. Nach dem Verfahren der vorliegenden Erfindung lassen sich diese Micellen mit den eingeschlossenen anorganischen und organischen Wirkstoffen bei pH = 7,0 auf einfache Weise ohne großen apparativen Aufwand in wässriger Phase herstellen, welche kleine, einfache Micellen mit einem Durchmesser von ca. 50-100 Å und große Micellen mit einem Durchmesser von 600-10.000 Å - je nach Gegenion - enthalten. Außerdem werden durch ein Gemisch von Glycerol/Ethanol im Verhältnis von 2 Gew.% : 15 Gew.% gegenüber Wasser beide Micellen verschiedener Größenordnung sowohl in ihrer Form (Kugel, Hemizylinder, Stab, Diskus) als auch in ihrer Kompaktheit durch Senkung der KMK, wie auch durch Reduktion der Freien Energie der gesamten Micelle in der wäßrigen Phase infolge Verdünnung der Elektronendichte an der externen Oberfläche, stabilisiert. Mittels geeigneter Trennmethoden, z.B. HPLC, Ultrafiltration, Gelfiltration und/oder präparativer Zentrifugation kann man kleine von großen Micellen präparativ trennen.

6. Die Stabilität, Haltbarkeit und Lagerfähigkeit dieser so hergestellten Micellen aus quartären organischen Ammoniumbasen, pH = 7,0, gegenüber Temperatur, Dichtigkeit ("sealing and leaking") und Lagerfähigkeit ist durch die Einlagerung der pharmazeutischen Wirkstoffe in hydrophoben Kern der Micellen im Verhältnis zu den Micellen ohne Wirkstoffe bei gleichen $Y^-$ erhöht. Im Gegensatz zu den Liposomen tritt hier kein Schmelzen bei höherer Temperatur (>40°C) ein, sondern bei verfahrensgemäßer Herstellung ändern sich die hydrodynamischen Verhältnisse erst ab >60°C. Da bei zunehmender Temperatur diese so hergestellten Micellen von quartären Ammoniumbasen eher eine Reduzierung im hydrodynamischen Radius eingehen, daher kompakter werden, sind diese Art Micellen thermodynamisch stabiler als künstliche Liposomen oder Liposomen + quartäre Ammoniumbasen. Diese Vorgänge sind leicht im Routine-Verfahren durch inelastische Lichtstreuung bei der Herstellung zu prüfen.

7. Die Hydrophobizität bzw. die Penetration der $H_2O$-Moleküle in diese so hergestellten Micellen und deren Beeinflussung durch anorganische pharmazeutische Wirkstoffe, z.B. $Hg(CN)_2$, ZnEDTA, $ZnSO_4$, ZnO, Wolframsäure-antimonate, $K_{18}(KW_{21}Sb_9O_{86})_{17}$, als auch der organischen Substanzen läßt sich durch NMR-Spektroskopie nachweisen.

## Patentansprüche

1. N-alkylierte quartäre stickstoffhaltige Heterozyklen der allgemeinen Formel

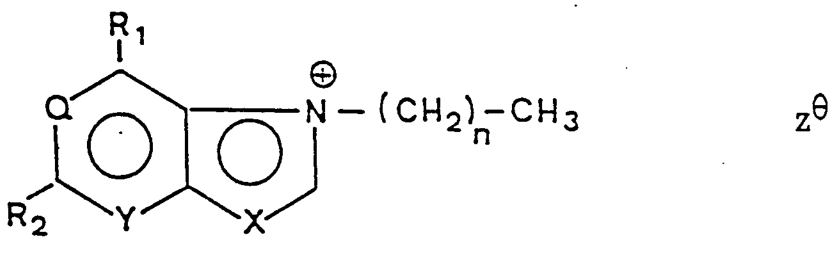

| | |
|---|---|
| X | = NH ; S |
| Y | = N ; CH |
| Q | = N ; C-$CH_3$ ; CH |
| $R_1$ | = H ; OH ; F ; $NH_2$ |
| $R_2$ | = H ; OH ; $NH_2$ |
| n | = 8 bis 20 insbesondere 15 und |
| $Z^-$ | = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$, ausgenommen die folgenden Verbindungen: 7-Dodecylbenzthiazoliumiodid und -bromid, N-Octadecylbenzthiazoliumiodid, Cetylbenzimidazoliumchlorid, 7-Dodecylchloridhydroxymethylbenzimidazoliumchlorid, 1-n-Decylbenzimidazoliumbromid, 1-n-Octyl-benzimidazoliumbromid, 1-Dodecylbenzimidazoliumchlorid. |

**2.** 7-n-Alkyl-imidazolium [4,5-d]-pyrimidin nach Anspruch 1 der Formel

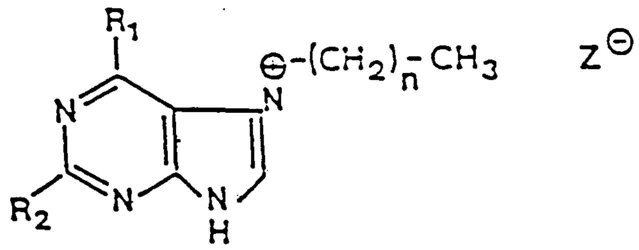

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$,
wobei $Z^-$ die Anionen gemäß dem Patentanspruch 1 und n 8 - 20 bedeuten.

**3.** 7-Hexadecylimidazolium [4,5-d]-pyrimidin nach Anspruch 1 oder 2 der Formel

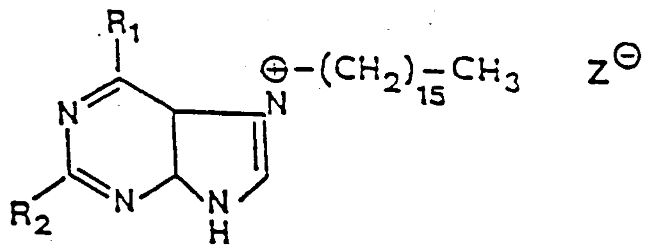

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$,
wobei $Z^-$ die Anionen gemäß dem Patentanspruch 1 bedeuten.

**4.** 3-n-Alkyl-5,6-substituierte Benzimidazolium-Verbindungen der Formel

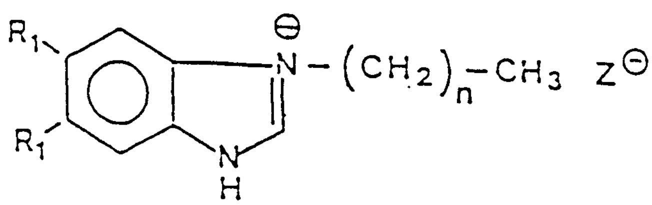

$R_1$ = OH
wobei $Z^-$ die Anionen gemäß dem Patentanspruch 1 und n 8 - 20 bedeuten.

**5.** 3-n-Alkyl-5,6-substituierte Benzthiazolium-Verbindungen der Formel

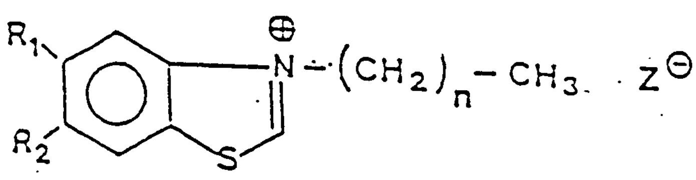

$R_1 = R_2 = OH$
$R_1 = R_2 = CH_3$
wobei $Z^-$ die Anionen gemäß dem Patentanspruch 1 und n 8 - 20 bedeuten.

**6.** Verfahren zur Herstellung der N-alkylierten quartären stickstoffhaltigen Heterozyklen nach einem oder mehreren der Ansprüche 1 bis 5,
dadurch gekennzeichnet,
daß der zu alkylierende Heterozyklus in einem geeigneten Lösungsmittel gelöst wird, anschließend unter stetem Rühren die Stöchiometrische Menge n-Alkyl-halogenid zugegeben wird, anschließend unter stetem Rühren am Rückfluß über eine geraume Zeit erhitzt wird, wonach das Endprodukt nach dem Abkühlen ausfällt.

**7.** Verfahren zur Herstellung der N-alkylierten quartären stickstoffhaltigen Heterozyklen nach einem oder mehreren der Ansprüche 1 bis 5,
dadurch gekennzeichnet,
daß der zu alkylierende, unsubstituierte Heterozyklus in einem geeigneten Lösungsmittel gelöst wird, die stöchiometrische Menge n-Alkyl-halogenid zugegeben wird, anschließend unter Druck bei 100°C für eine Reaktionsdauer von 8 Stunden die Reaktion vollendet wird, wonach das Endprodukt nach dem Abhkühlen ausfällt.

**8.** Verfahren zur Herstellung der N-alkylierten quartären stickstoffhaltigen Heterozyklen nach einem oder mehreren der Ansprüche 1 bis 5,
dadurch gekennzeichnet,
daß die zu alkylierenden Verbindungen des Purins in einem Lösungsmittel gelöst werden, die stöchiometrische Menge von n-Alkyl-magnesium-halogeniden in einem geeigneten Lösungsmittel unter stetem Rühren zugetropft wird, auf 40 - 80°C erwärmt wird und weiter gerührt wird für 12 Stunden, und die Reaktion durch Zugabe von 50 gew.%-iger Bromwasserstoffsäure beendet wird.

**9.** Verfahren zur Herstellung nach einem oder mehreren der Ansprüche 6 bis 8,
dadurch gekennzeichnet,
daß das Lösungsmittel 1,2-Dimethoxy-ethan und/oder n-Heptan ist.

**10.** Verfahren zur Herstellung der N-alkylierten quartären stickstoffhaltigen Heterozyklen nach einem oder mehreren der Ansprüche 1 bis 5,
dadurch gekennzeichnet,
daß die zu alkylierenden Verbindungen der Purine in einem geeigneten Lösungsmittel gelöst werden, unter gleichzeitiger Zugabe von Triethyloxonium-borfluorid ($Et_3OBF_4$) und das entsprechende n-Alkyl-halogenid, das ebenfalls in einem geeigneten Lösungsmittel gelöst ist, unter stetem Rühren zugetropft wird, und die Reaktion unter stetem Rühren bei 65 - 80°C nach 16 Stunden beendet wird, wonach das Reaktionsprodukt nach dem Abkühlen ausfällt.

**11.** Verfahren zur Herstellung nach Anspruch 10,
dadurch gekennzeichnet,
daß das Lösungsmittel Azeton oder Di-iso-butyl-keton oder Iso-butyl-ethyl-keton ist.

**12.** Verfahren zur Herstellung nach einem oder mehreren der Ansprüche 6 bis 11,
dadurch gekennzeichnet,
daß entweder das Reaktionsgefäß ein Reaktionskolben mit Rührwerk, Zutropfvorrichtung und Heizungseinrichtung ist oder das Reaktionsgefäß ein Edelstahl-Autoklav mit Druck- und Temperatur-Anzeige ist.

**13.** Verwendung der stickstoffhaltigen Heterozyklen der allgemeinen Formel

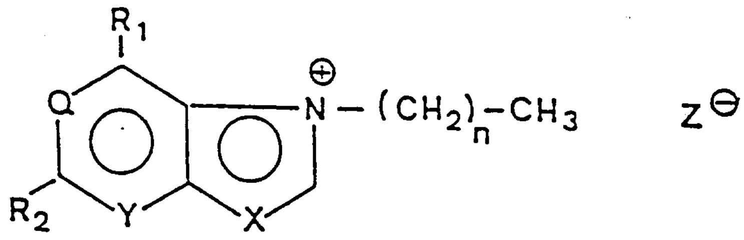

X = NH ; S
Y = N ; CH
Q = N ; C-$CH_3$ ; CH
$R_1$ = H ; OH ; F ; $NH_2$
$R_2$ = H ; OH ; $NH_2$
n = 8 bis 20 insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$

und der Verbindungen nach Anspruch 4 und 5 zur Herstellung von micellaren oder vesikulären Strukturen in polaren oder unpolaren Lösungsmitteln, insbesondere mit extrem niedriger KMK.

**14.** Verwendung nach Anspruch 13,
dadurch gekennzeichnet,
daß der Heterozyklus eine Verbindung der Ansprüche 2 bis 5 ist.

**15.** Pharmazeutische Zubereitung,
dadurch gekennzeichnet,
daß sie eine oder mehrere N-alkylierte quartäre stickstoffhaltige Heterozyklen der allgemeinen Formel

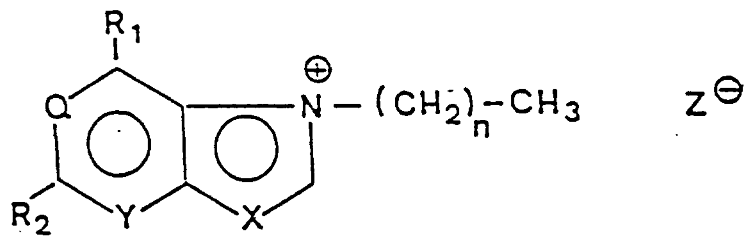

X = NH ; S
Y = N ; CH
Q = N ; C-$CH_3$ ; CH
$R_1$ = H ; OH ; F ; $NH_2$
$R_2$ = H ; OH ; $NH_2$
n = 8 bis 20 insbesondere 15 und
$Z^-$ = Chlorid, Bromid, Jodid, Maleat, Formiat, Acetat, Propionat, Hydrogensulfat, Malat, Fumarat, Salizylat, Alginat, Glukonat, Glukoronat, Galaktoronat, Ethylsulfat oder Hydrogenphosphat $H_2PO_4^-$ und/oder eine oder mehrere der Verbindungen nach Anspruch 4 und/oder 5

enthält, in die hydrophobe pharmazeutische Wirkstoffe bei pH 6-8 in polaren oder unpolaren Lösungsmitteln micellar oder vesikulär eingeschlossen sind.

**16.** Pharmazeutische Zubereitung, dadurch gekennzeichnet, daß sie eine oder mehrere der Heterozyklen aus Anspruch 15, 4 und 5 bei pH 6-8 in polaren Lösungsmitteln als eigenständige pharmazeutische Wirkstoffe enthält.

**17.** Pharmazeutische Zubereitung zur Therapie von Magenulcera, dadurch gekennzeichnet, daß sie einen oder mehrere der Heterozyklen der Ansprüche 4 und 5 als eigenständige pharmazeutische Wirkstoffe enthält.

**18.** Pharmazeutische Zubereitung nach einem der Ansprüche 15 bis 16, dadurch gekennzeichnet, daß sie als Heterozyklus eine Verbindung der Ansprüche 2 bis 5 enthält.

**19.** Verwendung der stickstoffhaltigen Heterozyklen aus Anspruch 4, 5 und 15 als spektroskopische Marker (Reporter-Gruppen) in immunologischen und klinisch-biochemischen Analyseverfahren sowie zu kolloid-chemischen Meßverfahren zur Bestimmung der KMK.

**20.** Verwendung nach Anspruch 19, dadurch gekennzeichnet, daß der Heterozyklus eine Verbindung der Ansprüche 2 bis 5 ist.

**Claims**

**1.** N-alkylated quaternary nitrogen-containing heterocycles of the general formula

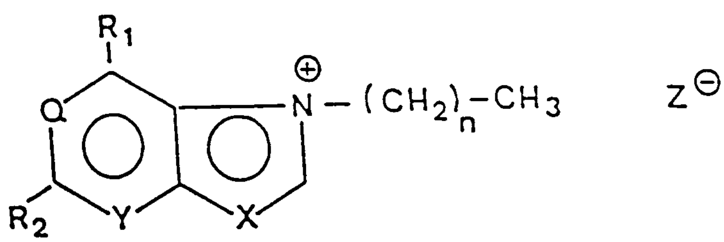

| | |
|---|---|
| X | = NH ; S |
| Y | = N ; CH |
| Q | = N ; C-$CH_3$ ; CH |
| $R_1$ | = H ; OH ; F ; $NH_2$ |
| $R_2$ | = H ; OH ; $NH_2$ |

wherein

| | |
|---|---|
| n | = 8 to 20, in particular 15 and |
| $z^-$ | = chloride, bromide, iodide, maleate, formate, acetate, propionate, hydrogensulfate, malate, fumarate, salicylate, alginate, gluconate, glucoronate, galactoconate, ethylsulfate or hydrogenphosphate $H_2PO_4^-$, |

with the proviso that the following compounds are excluded:
7-dodecyl benzthiazolium iodide and bromide;
N-octadecyl benzthiazolium iodide;
cetyl benzimidazolium chloride;
7-dodecyl chloride hydroxy methyl benzimidazolium chloride;
1-n-decyl imidazolium bromide;
1-n-octyl benzimidazolium bromide;
1-dodecyl benzimidazolium chloride.

**2.** 7-n-alkylimidazolium [4,5-d] -pyrimidine of the formula

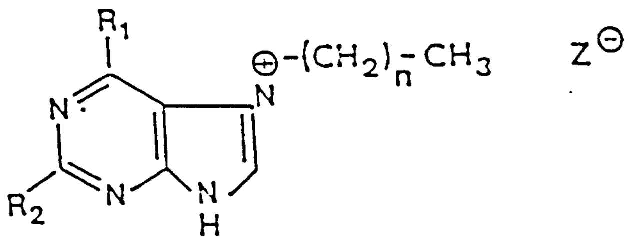

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$
wherein $Z^-$ denotes the anions according to claim 1 and n is 8 - 20.

**3.** 7-hexadecylimidazolium [4,5-d] pyrimidine of the formula

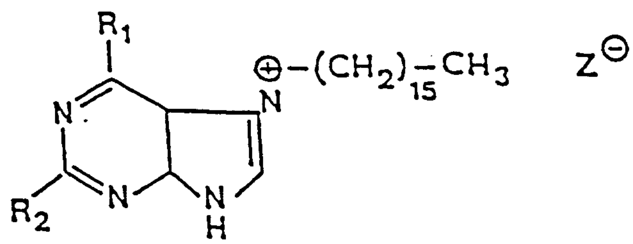

$R_1$ = OH ; $R_2$ = OH
$R_1$ = H ; $R_2$ = H
$R_1$ = F ; $R_2$ = $NH_2$
$R_1$ = F ; $R_2$ = OH
$R_1$ = $NH_2$ ; $R_2$ = H
$R_1$ = $NH_2$ ; $R_2$ = $NH_2$
wherein $Z^-$ denotes the anions according to claim 1

**4.** 3-n-alkyl-5,6-substituted benzimidazolium compounds of the formula

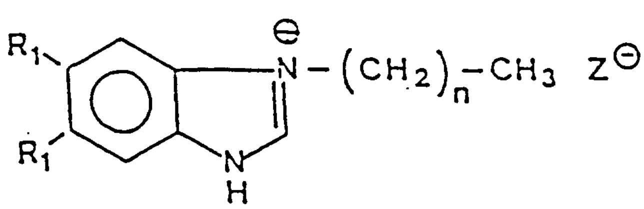

$R_1$ = OH
wherein $Z^-$ denotes the anions according to claim 1 and n is 8 - 20.

**5.** 3-n-alkyl-5,6-substituted benzthiazolium compounds of the formula

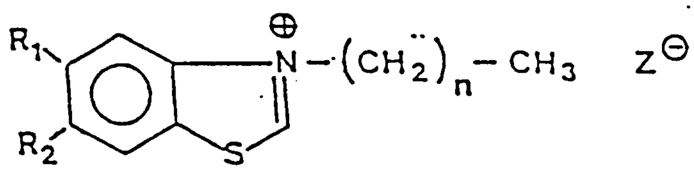

$R_1 = R_2 = OH$
$R_1 = R_2 = CH_3$
wherein $Z^-$ denotes the anions according to claim 1 and n is 8 - 20.

**6.** Process for the preparation of the N-alkylated quaternary nitrogen-containing heterocycles according to one or more of claims 1 - 5, characterized in that the heterocycle to be alkylated is dissolved in a suitable solvent thereafter with constant stirring the stoichiometric amount of n-alkyl halide is added, and thereafter with constant stirring reflux heating is carried out for a considerable period, whereupon the end product precipitates after cooling.

**7.** Process according to one or more of claims 1 - 5, characterized in that the unsubstituted heterocycle to be alkylated is dissolved in a suitable solvent, the stoichiometric amount of n-alkyl halide is added, and thereafter under pressure at 100°C the reaction is completed for a reaction duration or 8 hours, whereupon the end product precipitates after cooling.

**8.** Process for the preparation according to one or more of claims 1 - 5, characterized in that the compounds of purine to be alkylated are dissolved in a solvent, the stoichiometric amount of n-alkylmagnesium halides in a suitable solvent is added dropwise with constant stirring, heating carried out to 40 - 80°C and further stirring for 12 hours and the reaction is terminated by addition of a 50% by weight solution of hydrobromic acid.

**9.** Process for the preparation according to one or more of claims 6 - 8, characterized in that the solvent is 1,2-dimethoyyethane and/or n-heptane.

**10.** Process for the preparation according to one or more of claims 1 - 5, characterized in that the compounds to be alkylated of the purines are dissolved in a suitable solvent, with simultaneous addition of triethyloxonium boron tetra fluoride ($Et_3OBF_4$) and the corresponding n-alkyl halide, which is also dissolved in a suitable solvent, dropwise with constant stirring, and the reaction with constant stirring at 65 - 80°C is terminated after 16 hours, whereafter the reaction product precipitates after the cooling.

**11.** Process for the preparation according to claim 10, characterized in that the solvents are acetone or disobutylketone or isobutylethylketone.

**12.** Process for the preparation according to one or more of claims 6-11, characterized in that the reaction vessel is either a reaction flask with stirring mechanism, an apparatus for dropwise addition and a heating means or the reaction vessel is a stainless steel autoclave with pressure and temperature indication.

**13.** Use of the nitrogen-containing heterocycles of the general formula

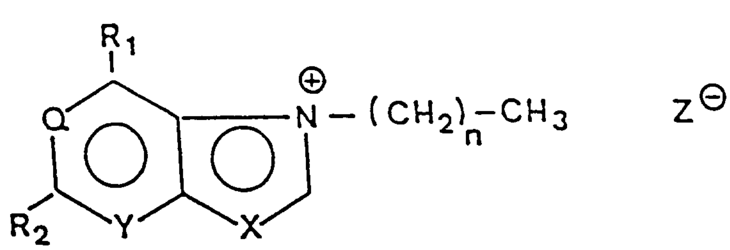

| | |
|---|---|
| X | = NH ; S |
| Y | = N ; CH |
| Q | = N ; C-$CH_3$ ; CH |
| $R_1$ | = H ; OH ; F ; $NH_2$ |
| $R_2$ | = H ; OH ; $NH_2$ |

wherein

| | |
|---|---|
| n | = 8 to 20, in particular 15 and |
| $z^-$ | = chloride, bromide, iodide, maleate, formate, acetate, propionate, hydrogensulfate, malate, fumarate, salicylate, alginate, gluconate, glucoronate, galactoronate, ethylsulfate or hydrogenphosphate $H_2PO_4^-$, |

and the compounds according to claim 4 and 5 for preparing micellar or vesicular structures in polar or nonpolar solvents, in particular with extremely low cmc.

**14.** Use according to claim 13, characterized in that the heterocycle is a compound according to one of the claims 2 - 5.

**15.** Pharmaceutical preparation characterized in that it contains one or more of N-alkylated quaternary nitrogen containing heterocycles of the general formula

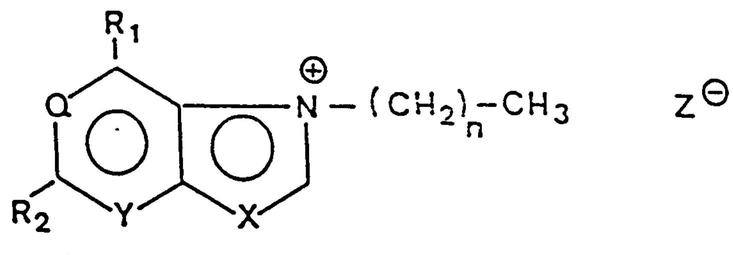

| | |
|---|---|
| X | = NH ; S |
| Y | = N ; CH |
| Q | = N ; C-$CH_3$ ; CH |
| $R_1$ | = H ; OH ; F ; $NH_2$ |
| $R_2$ | = H ; OH ; $NH_2$ |

wherein

| | |
|---|---|
| n | = 8 to 20, in particular 15 and |
| $z^-$ | = chloride, bromide, iodide, maleate, formate, acetate, propionate, hydrogensulfate, malate, fumarate, salicylate, alginate, gluconate, glucoronate, galactoronate, ethylsulfate or hydrogenphosphate $H_2PO_4^-$, |

and/or one or more of the compounds according to claim 4 and/or 5 wherein hydrophobic pharmaceutically active compounds are included in micelles or vesicles at pH 6 - 8 in polar or nonpolar solvents.

**16.** Pharmaceutical preparation characterized in that it contains one or more of the heterocycles according to claim 15, 4 and 5 at pH 6 - 8 in polar solvents as independent pharmaceutically active substances.

**17.** Pharmaceutical preparation for the therapy of gastric ulcers characterized in that it contains one or more of the heterocycles according to one of the claims 4 and 5 as independent pharmaceutically active substances.

18. Pharmaceutical preparation according to one of the claims 15 to 16 characterized in that it contains a compound according to claims 2 - 5 as a heterocycle.

19. Use of the nitrogen-containing heterocycles according to one of the claims 4, 5 and 15 as spectroscopic markers (reporter groups) in immunological and clinical-biochemical analysis methods and for colloidal chemistry measuring methods for determining the cmc.

20. Use according to claim 19, characterized in that the heterocycle is a compound according to claims 2 to 5.

**Revendications**

1. Hétérocycles à atome d'azote quaternaire, N-alkylés, de formule générale

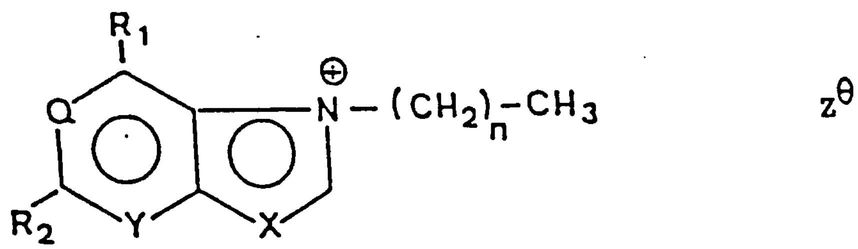

dans laquelle

X = NH, S
Y = N, CH
Q = N, C-$CH_3$, CH
$R_1$ = H, OH, F, $NH_2$
$R_2$ = H, OH, $NH_2$
n = 8 à 20, en particulier 15 et
$Z^-$ représente l'anion chlorure, bromure, iodure, maléate, formiate, acétate, propionate, hydrogénosulfate, malate, fumarate, salicylate, alginate, gluconate, glucuronate, galacturonate, éthylsulfate ou hydrogénophosphate $H_2PO_4^-$,

à l'exception des composés suivants:
iodure et bromure de 7-dodécylbenzothiazolium, iodure de N-octadécylbenzothiazolium, chlorure de cétylbenzimidazolium, chlorure de 7-dodécylchlorhydroxyméthylbenzimidazolium, bromure de 1-n-décylbenzimidazolium, bromure de 1-n-octylbenzimidazolium, chlorure de 1-dodécylbenzimidazolium.

2. 7-n-alkylimidazolium[4,5-d]pyrimidine selon la revendication 1, de formule

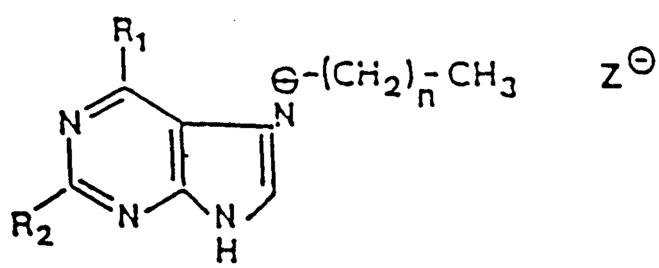

$R_1$ = OH ; $R_2$ = OH
$R_1$ = H ; $R_2$ = H
$R_1$ = F ; $R_2$ = $NH_2$
$R_1$ = F ; $R_2$ = OH

$R_1 = NH_2$ ; $R_2 = H$
$R_1 = NH_2$ ; $R_2 = NH_2$,
Z' représentant les anions selon la revendication 1 et n allant de 8 à 20.

**3.** 7-hexadécylimidazolium[4,5-d]pyrimidine selon la revendication 1 ou 2, de formule

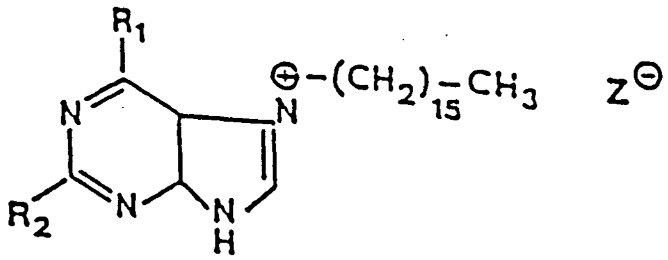

$R_1 = OH$ ; $R_2 = OH$
$R_1 = H$ ; $R_2 = H$
$R_1 = F$ ; $R_2 = NH_2$
$R_1 = F$ ; $R_2 = OH$
$R_1 = NH_2$ ; $R_2 = H$
$R_1 = NH_2$ ; $R_2 = NH_2$,
Z' représentant les anions selon la revendication 1.

**4.** Composés benzimidazolium 3-n-alkyl-5,6-substitués de formule

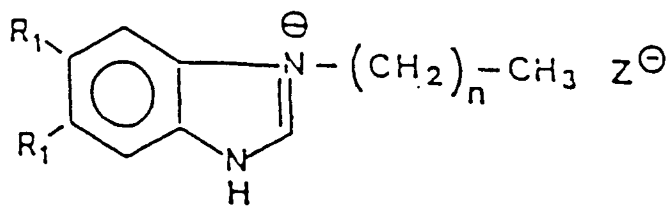

$R_1 = OH$
Z' représentant les anions selon la revendication 1, et n allant de 8 à 20.

**5.** Composés benzothiazolium 3-n-alkyl-5,6-substitués de formule

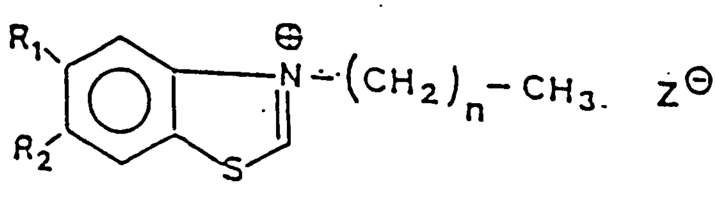

$R_1 = R_2 = OH$
$R_1 = R_2 = CH_3$
Z' représentant les anions selon la revendication 1, et n allant de 8 à 20.

**6.** Procédé pour la préparation des hétérocycles à atome d'azote quaternaire N-alkylés, selon une ou plusieurs des revendications 1 à 5, caractérisé en ce que l'on dissout dans un solvant approprié l'hétérocycle à alkyler, puis on y ajoute, sous constante agitation, la quantité stoechiométrique d'halogénure de n-alkyle, et ensuite on chauffe le mélange au reflux sous constante agitation, pendant un grand intervalle de temps, à la suite de quoi le produit final précipite après refroidissement.

7. Procédé pour la préparation d'hétérocycles à atome d'azote quaternaire N-alkylés, selon une ou plusieurs des revendications 1 à 5, caractérisé en ce que l'on dissout dans un solvant approprié l'hétérocycle non substitué à alkyler, on y ajoute la quantité stoechiométrique d'halogénure de n-alkyle, et on poursuit ensuite la réaction jusqu'à son achèvement, à 100°C, sous pression, pendant une durée de réaction de 8 heures, à la suite de quoi le produit final précipite après refroidissement.

8. Procédé pour la préparation des hétérocycles à atome d'azote quaternaire N-alkylés selon une ou plusieurs des revendications 1 à 5, caractérisé en ce que l'on dissout dans un solvant les dérivés à alkyler de la purine, on y ajoute goutte à goutte, sous constante agitation, la quantité stoechiométrique d'halogénures de n-alkylmagnésium dans un solvant approprié, on chauffe à 40-80°C et on poursuit l'agitation pendant 12 heures, puis on met fin à la réaction par addition d'acide bromhydrique à 50 % en poids.

9. Procédé pour la préparation selon une ou plusieurs des revendications 6 à 8, caractérisé en ce que le solvant est le 1,2-diméthoxyéthane et/ou le n-heptane.

10. Procédé pour la préparation des hétérocycles à atome d'azote quaternaire N-alkylés selon une ou plusieurs des revendications 1 à 5, caractérisé en ce que l'on dissout dans un solvant approprié des dérivés à alkyler de la purine, avec addition simultanée de borofluorure de triéthyloxonium ($Et_3OBF_4$) et on y ajoute goutte à goutte, sous constante agitation, l'halogénure de n-alkyle correspondant, qui est également dissous dans un solvant convenable, et la réaction est achevée sous constante agitation, à 65-80°C, au bout de 16 heures, à la suite de quoi le produit de réaction précipite après refroidissement.

11. Procédé pour la préparation selon la revendication 10, caractérisé en ce que le solvant est l'acétone ou la diisobutylcétone ou l'isobutyléthylcétone.

12. Procédé pour la préparation selon une ou plusieurs des revendications 6 à 11, caractérisé en ce que soit le récipient de réaction est un ballon de réaction muni d'agitateur, d'un dispositif d'addition goutte à goutte et d'un dispositif de chauffage, soit le récipient de réaction est un autoclave en acier spécial, muni d'indicateur de pression et de température.

13. Utilisation des hétérocycles azotés de formule générale

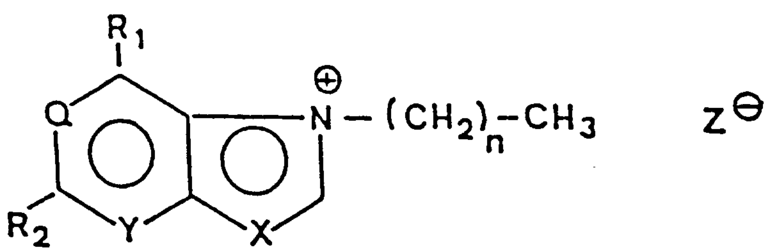

dans laquelle

| | |
|---|---|
| X | = NH, S |
| Y | = N, CH |
| Q | = N, C-$CH_3$, CH |
| $R_1$ | = H, OH, F, $NH_2$ |
| $R_2$ | = H, OH, $NH_2$ |
| n | = 8 à 20, en particulier 15 et |
| $Z^-$ | représente l'anion chlorure, bromure, iodure, maléate, formiate, acétate, propionate, hydrogénosulfate, malate, fumarate, salicylate, alginate, gluconate, glucuronate, galacturonate, éthylsulfate ou hydrogénophosphate $H_2PO_4^-$, |

et des composés selon les revendications 4 et 5, pour la préparation de structures micellaires ou vésiculaires dans des solvants polaires ou non polaires, en particulier à extrêmement basse concentration critique de formation de micelles (CCM).

14. Utilisation selon la revendication 13, caractérisée en ce que l'hétérocycle est un composé des revendications 2 à 5.

**15.** Composition pharmaceutique, caractérisée en ce qu'elle contient un ou plusieurs hétérocycles à atome d'azote quaternaire N-alkylés de formule générale

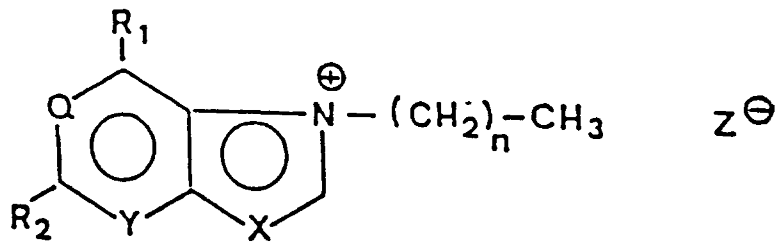

dans laquelle

X = NH, S
Y = N, CH
Q = N, C-$CH_3$, CH
$R_1$ = H, OH, F, $NH_2$
$R_2$ = H, OH, $NH_2$
n = 8 à 20, en particulier 15 et
$Z^-$ représente l'anion chlorure, bromure, iodure, maléate, formiate, acétate, propionate, hydrogénosulfate, malate, fumarate, salicylate, alginate, gluconate, glucuronate, galacturonate, éthylsulfate ou hydrogénophosphate $H_2PO_4^-$,

et/ou un ou plusieurs des composés selon la revendication 4 et/ou la revendication 5, dans lesquels des substances actives pharmaceutiques hydrophobes sont incluses sous forme micellaire ou vésiculaire à pH 6-8, dans des solvants polaires ou non polaires.

**16.** Composition pharmaceutique, caractérisée en ce qu'elle contient un ou plusieurs des hétérocycles des revendications 15, 4 et 5, à pH 6-8 dans des solvants polaires, en tant que substances pharmaceutiques autonomes.

**17.** Composition pharmaceutique pour le traitement d'ulcères gastriques, caractérisée en ce qu'elle contient un ou plusieurs des hétérocycles des revendications 4 et 5, en tant que substances actives pharmaceutiques autonomes.

**18.** Composition pharmaceutique selon la revendication 15 ou 16, caractérisée en ce qu'elle contient, en tant qu'hétérocycle, un composé des revendications 2 à 5.

**19.** Utilisation des hétérocycles azotés des revendications 4, 5 et 15, en tant que marqueurs spectroscopiques (groupes reporter) dans des procédés d'analyses immunologiques et cliniques-biochimiques ainsi que dans des procédés de mesure de la chimie des colloïdes, pour la détermination de la CCM.

**20.** Utilisation selon la revendication 19, caractérisée en ce que l'hétérocycle est un composé des revendications 2 à 5.